(19) Europäisches Patentamt
European Patent Office
Office européen des brevets

(11) Veröffentlichungsnummer: **0 382 112 A2**

# (12) EUROPÄISCHE PATENTANMELDUNG

(21) Anmeldenummer: 90102067.7

(22) Anmeldetag: 02.02.90

(51) Int. Cl.5: C07D 239/62, C07D 239/54, C07D 405/12, C07D 401/12, C07D 239/66, A01N 43/54

Patentanspruch für folgenden Vertragsstaat: ES.

(30) Priorität: 06.02.89 DE 3903404

(43) Veröffentlichungstag der Anmeldung: 16.08.90 Patentblatt 90/33

(84) Benannte Vertragsstaaten: AT BE CH DE DK ES FR GB GR IT LI NL SE

(71) Anmelder: HOECHST AKTIENGESELLSCHAFT
Postfach 80 03 20
D-6230 Frankfurt am Main 80(DE)

(72) Erfinder: Kratt, Günter, Dr.
Wiesenstrasse 18
D-6239 Eppstein / Taunus(DE)
Erfinder: Salbeck, Gerhard, Dr.
Königsberger Strasse 52
D-6239 Kriftel / Taunus(DE)
Erfinder: Bonin, Werner, Dr.
Im Schulzehnten 18
D-6233 Kelkheim (Taunus)(DE)
Erfinder: Düwel, Dieter, Dr.
Frankfurter Strasse 39
D-6238 Hofheim am Taunus(DE)

(54) **Pyrimidintrionderivate, Verfahren zu ihrer Herstellung, sie enthaltende Mittel und ihre Verwendung als Schädlingsbekämpfungsmittel.**

(57) Verbindungen der Formel I, deren tautomere Formen der Formel Ia,deren Stereoisomere sowie die Gemische dieser Formen

EP 0 382 112 A2

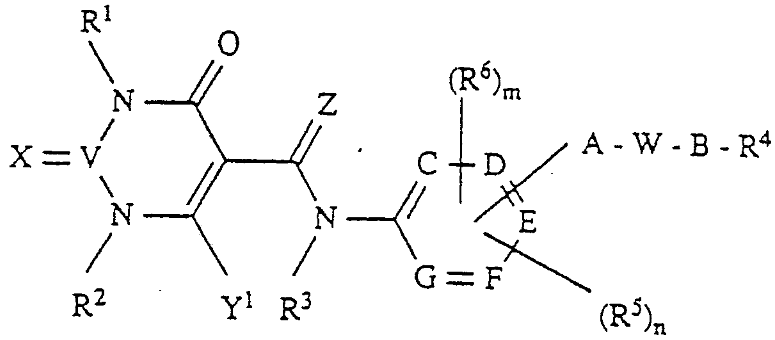

(I) ,

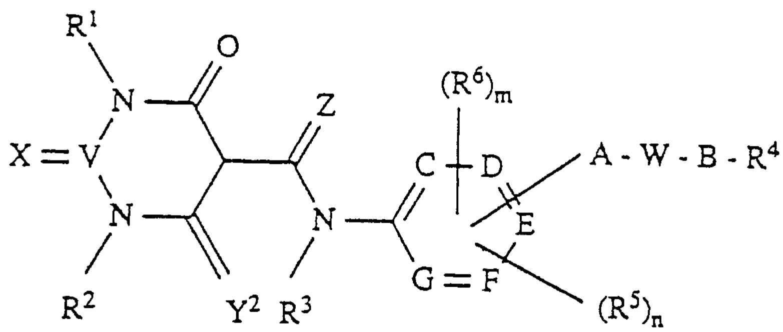

(Ia) ,

worin

$R^1$,$R^2$ unsubstituiertes oder substituiertes Alkyl, Alkenyl, Alkinyl; Alkoxy, Alkylsulfonyl, Alkylcarbonyl, Cyano oder gegebenenfalls substituiertes Phenyl, Phenylsulfonyl oder Phenylcarbonyl,

$R^3$ Wasserstoff, Alkyl oder gegebenenfalls substituiertes Phenyl,

$R^4$ H, OH, unsubstituiertes oder substituiertes Alkyl, Alkenyl, Alkinyl, Phenylsulfonyl, Phenylcarbonyl, Phenylcarbamoyl oder Phenyl; Alkylsulfonyl, Alkylcarbonyl, die halogeniert sein können, Alkylcarbamoyl oder Cyano,

$R^5$, $R^6$ Halogen, Alkyl, Alkoxy, Alkylcarbonyl, Alkoxycarbonyl, Alkylsulfenyl, -sulfinyl, -sulfonyl, Haloalkoxy, Alkylamino, Cyano, Sulfo, Nitro,

A,X,Z,B Sauerstoff, Schwefel, Sulfinyl, Sulfonyl oder Stickstoff der durch Alkyl substituiert sein kann, oder für B eine Doppelbindung,

C,D,E,F,G Kohlenstoff oder Stickstoff,

V Kohlenstoff oder substituierten Phosphor,

W Alkylen oder Haloalkylen, die beide substituiert sein können,

$Y^1$ H, Halogen, Alkyl, Alkylamino oder Hydroxy,

$Y^2$ Sauerstoff, Schwefel oder gegebenenfalls durch Alkyl substituierten Stickstoff und

n,m 0-4 bedeuten, sowie deren in der Landwirtschaft einsetzbaren oder physiologisch verträglichen Salze, besitzen vorteilhafte Wirkungen gegen ein breites Spektrum tierischer Schädlinge in der Landwirtschaft und der Tierzucht.

## Pyrimidintrionderivate, Verfahren zu ihrer Herstellung, sie enthaltende Mittel und ihre Verwendung als Schädlingsbekämpfungsmittel

Barbitursäurederivate mit insektizider bzw. anthelminthischer Wirksamkeit sind aus der EP-A 102327 bekannt. Diese besitzen jedoch teilweise unzureichende Wirksamkeiten.

Es wurden neue substituierte Pyrimidintrione gefunden, die sich durch vorteilhafte Eigenschaften bei der Bekämpfung von Schädlingen wie z.B. Schadinsekten oder Endoparasiten auszeichnen.

Gegenstand der vorliegenden Erfindung sind daher die Verbindungen der Formel I, deren tautomere Formen der Formel Ia, deren Stereoisomere sowie die Gemische dieser Formen

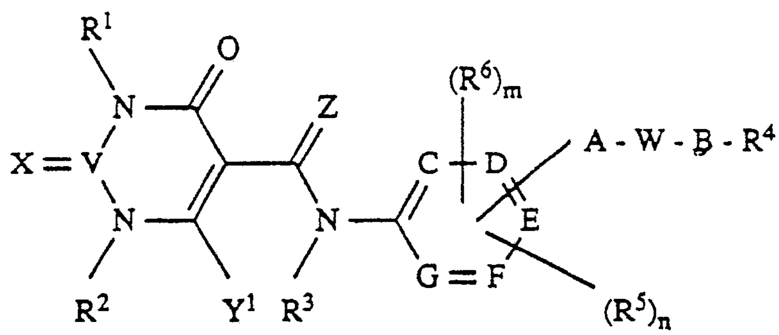

(I),

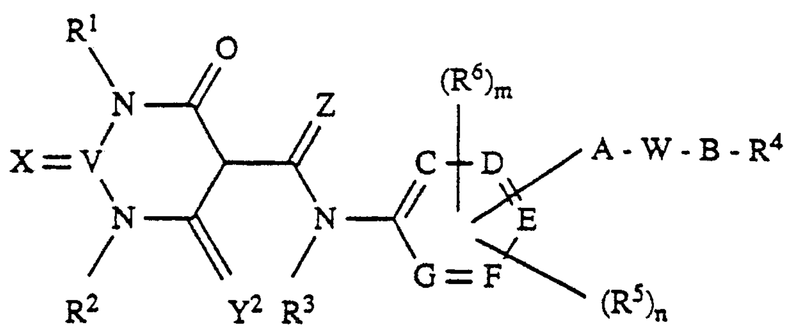

(Ia),

worin

$R^1$,$R^2$ unabhängig voneinander, ($C_1$-$C_6$)-Alkyl, ($C_2$-$C_6$)-Alkenyl, ($C_2$-$C_6$)-Alkinyl, die alle durch ein bis dreizehn Halogenatome und/oder ein bis dreifach durch einen Rest der Gruppe ($C_1$-$C_4$)-Alkoxy, ($C_1$-$C_4$)-Alkoxycarbonyl, ($C_2$-$C_4$)-Alkylsulfenyl, ($C_1$-$C_4$)-Alkylsulfinyl, ($C_1$-$C_4$)-Alkylsulfonyl, Di($C_1$-$C_4$)-Alkylamino, Cyano, Sulfo, Nitro, Hydroxy, Carboxy, Phenyl oder Phenoxy, wobei die beiden letztgenannten Reste ein- bis dreifach durch einen Rest der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkoxy mit ein bis neun Halogenatomen, Di($C_1$-$C_4$)Alkylamino, Cyano, Sulfo, Nitro substituiert sein können, substituiert sein können; ($C_1$-$C_4$)-Alkoxy, ($C_1$-$C_4$)-Alkylsulfonyl, ($C_1$-$C_4$)-Alkylcarbonyl, Cyano, Phenylsulfonyl, Phenylcarbonyl, wobei die beiden letztgenannten Reste durch ein bis drei Reste der Gruppe Halogen, ($C_1$-$C_4$)-Alkyl, ($C_1$-$C_4$)-Alkoxy, Halo($C_1$-$C_4$)-alkoxy mit ein bis neun Halogenatomen oder Nitro substituiert sein können;

Phenyl, das durch in bis drei Reste aus der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo($C_1$-$C_4$)-alkoxy, mit jeweils ein bis neuen Halogenatomen, Di($C_1$-$C_4$)-Alkylamino, Cyano, Sulfo, Nitro oder Phenoxy, das gegebenenfalls durch ein bis drei Rest der Gruppe Halogen, ($C_1$-$C_4$)-Alkyl, ($C_1$-$C_4$)-Alkoxy substituiert ist, substituiert sein kann,

$R^3$ Wasserstoff, ($C_1$-$C_6$)-Alkyl; Phenyl, das durch ein bis drei Reste aus der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo($C_1$-$C_4$)-alkoxy, mit jeweils ein bis neun Halogenatomen, Di($C_1$-$C_4$)-Alkylamino, Cyano, Sulfo, Nitro oder Phenoxy, das gegebenenfalls durch ein bis drei Reste der Gruppe Halogen, ($C_1$-$C_4$)-Alkyl, ($C_1$-$C_4$)-Alkoxy substituiert ist, substituiert sein kann,

$R^4$ Wasserstoff, $(C_1\text{-}C_6)$-Alkyl, $(C_2\text{-}C_6)$-Alkenyl, $(C_2\text{-}C_6)$-Alkinyl, die durch ein- bis neun Halogenatome und/oder ein- bis dreifach durch einen Rest der Gruppe $(C_1\text{-}C_6)$-Alkoxy, Halo$(C_1\text{-}C_6)$-alkoxy, $(C_1\text{-}C_6)$-Alkoxycarbonyl, Halo$(C_1\text{-}C_6)$-alkoxycarbonyl, $(C_1\text{-}C_6)$-Alkylsulfenyl, $(C_1\text{-}C_6)$-Alkylsulfinyl, $(C_1\text{-}C_6)$-Alkylsulfonyl, Di$(C_1\text{-}C_6)$-Alkylamino, Cyano, Sulfo, Nitro, Hydroxy, Carboxy, Phenyl oder Phenoxy, wobei die beiden letztgenannten Reste ein- bis dreifach durch inen Rest der Gruppe Halogen, $(C_1\text{-}C_4)$-Alkyl, Halo$(C_1\text{-}C_4)$-alkyl mit ein bis neun Halogenatomen, $(C_1\text{-}C_4)$-Alkoxy, $(C_1\text{-}C_4)$-Alkylcarbonyl, Halo$(C_1\text{-}C_4)$-alkylcarbonyl, $(C_1\text{-}C_4)$-Alkoxycarbonyl, Halo$(C_1\text{-}C_4)$-alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Sulfato, Halo$(C_1\text{-}C_4)$-alkoxy mit ein bis neun Halogenatomen, Di$(C_1\text{-}C_4)$-Alkylamino, Cyano, Sulfo, Nitro substituiert sein können, substituiert sein können; $(C_1\text{-}C_6)$-Alkylsulfonyl, $(C_1\text{-}C_6)$-Alkylcarbonyl, Halo$(C_1\text{-}C_6)$-alkylsulfonyl, Halo$(C_1\text{-}C_6)$-alkylcarbonyl, Mono-, Di$(C_1\text{-}C_4)$-Alkylcarbamoyl, Cyano, Phenylsulfonyl, Phenylcarbonyl, Phenylcarbamoyl, wobei die drei letztgenannten Rest durch ein bis drei Reste der Gruppe Halogen, $(C_1\text{-}C_4)$-Alkyl, Halo$(C_1\text{-}C_4)$-alkyl, $(C_1\text{-}C_4)$-Alkoxy, Halo$(C_1\text{-}C_4)$-alkoxy, $(C_1\text{-}C_4)$-Alkylcarbonyl, Halo-$(C_1\text{-}C_4)$-alkylcarbonyl, $(C_1\text{-}C_4)$-Alkoxycarbonyl, Halo$(C_1\text{-}C_4)$-alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Di$(C_1\text{-}C_4)$-Alkylamino, Cyano, Sulfo, Nitro substituiert sein können; Phenyl, das durch ein bis drei Reste aus der Gruppe Halogen, $(C_1\text{-}C_4)$-Alkyl, $(C_1\text{-}C_4)$-Alkoxy, $(C_1\text{-}C_4)$-Alkylcarbonyl, $(C_1\text{-}C_4)$-Alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Halo$(C_1\text{-}C_4)$-alkyl, Halo$(C_1\text{-}C_4)$-alkoxy, mit jeweils ein bis neun Halogenatomen, Di$(C_1\text{-}C_4)$-Alkylamino, Cyano, Sulfo, Nitro oder Phenoxy, das gegebenenfalls durch ein bis drei Reste der Gruppe Halogen, $(C_1\text{-}C_4)$-Alkyl, $(C_1\text{-}C_4)$-Alkoxy substituiert ist, substituiert sein kann,

$R^5$,$R^6$ unabhängig voneinander Halogen, $(C_1\text{-}C_4)$-Alkyl, $(C_1\text{-}C_4)$-Alkoxy, $(C_1\text{-}C_4)$-Alkylcarbonyl, $(C_1\text{-}C_4)$-Alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Halo$(C_1\text{-}C_4)$-alkyl, Halo-$(C_1\text{-}C_4)$-alkoxy mit jeweils ein bis neun Halogenatomen, Di$(C_1\text{-}C_4)$-Alkylamino, Cyano, Sulfo, Nitro,

A,X,Z unabhängig voneinander Sauerstoff, Schwefel, Sulfinyl, Sulfonyl oder Stickstoff, der durch $(C_1\text{-}C_4)$-Alkyl substituiert sein kann,

B Sauerstoff, Schwefel, Sulfinyl, Sulfonyl oder Stickstoff, der durch $(C_1\text{-}C_4)$-Alkyl substituiert sein kann; oder eine Doppelbindung,

C,D,E,F,G unabhängig voneinander Kohlenstoff oder Stickstoff,

V Kohlenstoff oder Phosphor, der durch $(C_1\text{-}C_6)$-Alkoxy, $(C_1\text{-}C_4)$-Alkylthio, Phenoxy oder Phenylthio, die beide ein bis drei Reste aus der Gruppe Halogen, $(C_1\text{-}C_4)$-Alkyl, $(C_1\text{-}C_4)$-Alkoxy, $(C_1\text{-}C_4)$-Alkylcarbonyl, $(C_1\text{-}C_4)$-Alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Halo$(C_1\text{-}C_4)$-alkyl, Halo$(C_1\text{-}C_4)$-alkoxy mit jeweils ein bis neun Halogenatomen, Di$(C_1\text{-}C_4)$-Alkylamino, Cyano, Sulfo, Nitro tragen können, substituiert ist,

W $(C_1\text{-}C_{10})$-Alkylen, Halo$(C_1\text{-}C_{10})$-Alkylen, die durch ein bis fünf Reste der Gruppe $(C_1\text{-}C_4)$-Alkyl, Halo-$(C_1\text{-}C_4)$-Alkyl, $(C_1\text{-}C_4)$-Alkoxy, Halo$(C_1\text{-}C_4)$-Alkoxy, Cyano oder Nitro substituiert sein können,

$Y^1$ H, Halogen, $(C_1\text{-}C_4)$-Alkyl, Amino, $(C_1\text{-}C_4)$-Alkylamino, Di$(C_1\text{-}C_4)$-Alkylamino, Hydroxy,

$Y^2$ Sauerstoff, Schwefel oder Stickstoff, der durch $(C_1\text{-}C_4)$-Alkyl substituiert sein kann, und

n,m unabhängig voneinander die Zahlen 0 bis 4 bedeuten, sowie deren in der Landwirtschaft einsetzbaren oder physiologisch verträglichen Salze.

Dabei bedeutet Alkyl geradkettiges oder verzweigtes Alkyl und Alkylen einen ungesättigten Kohlenwasserstoffrest der eine oder mehrere Doppelbindungen enthalten kann.

Bevorzugte Verbindungen der Formeln I und Ia sind solche, bei denen

$R^1$,$R^2$ unabhängig voneinander, $(C_1\text{-}C_6)$-Alkyl, $(C_2\text{-}C_6)$-Alkenyl, $(C_2\text{-}C_6)$-Alkinyl, die durch ein bis dreizehn Halogenatome und/oder ein bis dreifach durch einen Rest der Gruppe $(C_1\text{-}C_4)$-Alkoxy, $(C_1\text{-}C_4)$-Alkoxycarbonyl, $(C_1\text{-}C_4)$-Alkylsulfenyl, Di$(C_1\text{-}C_4)$-Alkylamino, Cyano, Sulfo, Nitro, Hydroxy, Carboxy, Phenyl oder Phenoxy, wobei die beiden letztgenannten Reste ein- bis dreifach durch einen Rest der Gruppe Halogen, $(C_1\text{-}C_4)$-Alkyl, $(C_1\text{-}C_4)$-Alkoxy, $(C_1\text{-}C_4)$-Alkylcarbonyl, $(C_1\text{-}C_4)$-Alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Halo$(C_1\text{-}C_4)$-alkoxy mit ein bis neun Halogenatomen, Di$(C_1\text{-}C_4)$-Alkylamino, Cyano, Sulfo, Nitro substituiert sein können, substituiert sein können,

$R^3$ Wasserstoff, $(C_1\text{-}C_6)$-Alkyl,

$R^4$ Wasserstoff, $(C_1\text{-}C_6)$-Alkyl,$(C_2\text{-}C_6)$-Alkenyl, $(C_2\text{-}C_6)$-Alkinyl, die durch ein- bis neun Halogenatome und/oder ein- bis dreifach durch einen Rest der Gruppe $(C_1\text{-}C_6)$-Alkoxy, Halo$(C_1\text{-}C_6)$-alkoxy, $(C_1\text{-}C_6)$-Alkoxycarbonyl, Halo$(C_1\text{-}C_6)$-alkoxycarbonyl, $(C_1\text{-}C_6)$-Alkylsulfenyl, Cyano, Sulfo, Nitro, Hydroxy, Carboxy, Phenyl oder Phenoxy, wobei die beiden letztgenannten Reste ein- bis dreifach durch einen Rest der Gruppe Halogen, $(C_1\text{-}C_4)$-Alkyl, Halo$(C_1\text{-}C_4)$-alkyl, $(C_1\text{-}C_4)$-Alkoxy, Halo$(C_1\text{-}C_4)$-alkoxy, $(C_1\text{-}C_4)$-Alkylcarbonyl, Halo$(C_1\text{-}C_4)$-alkylcarbonyl, $(C_1\text{-}C_4)$-Alkoxycarbonyl, Halo$(C_1\text{-}C_4)$-alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Di$(C_1\text{-}C_4)$-Alkylamino, Cyano, Sulfo, Nitro substituiert sein können, substituiert sein können;

$(C_1\text{-}C_6)$-Alkylsulfonyl, $(C_1\text{-}C_6)$-Alkylcarbonyl, Halo$(C_1\text{-}C_6)$-alkylsulfonyl, Halo$(C_1\text{-}C_6)$-alkylcarbonyl, Mono-, Di-

$(C_1-C_4)$-Alkylcarbamoyl, Cyano, Phenylsulfonyl, Phenylcarbonyl, Phenylcarbamoyl, wobei die drei letztgenannten Reste durch ein bis drei Reste der Gruppe Halogen, $(C_1-C_4)$-Alkyl, Halo$(C_1-C_4)$-alkyl, $(C_1-C_4)$-Alkoxy, Halo$(C_1-C_4)$-alkoxy, $(C_1-C_4)$-Alkylcarbonyl, Halo$(C_1-C_4)$-alkylcarbonyl $(C_1-C_4)$-Alkoxycarbonyl, Halo-$(C_1-C_4)$-alkoxycarbonyl, $(C_1-C_4)$-Alkylsulfenyl, $(C_1-C_4)$-Alkylsulfinyl, $(C_1-C_4)$-Alkylsulfonyl, Di$(C_1-C_4)$-Alkylamino, Cyano, Sulfo, Nitro substituiert sein können,

$R^5$,$R^6$ unabhängig voneinander Halogen, $(C_1-C_4)$-Alkyl, $(C_1-C_4)$-Alkoxy, $(C_1-C_4)$-Alkylcarbonyl, $(C_1-C_4)$-Alkoxycarbonyl, $(C_1-C_4)$-Alkylsulfenyl, Halo$(C_1-C_4)$-alkoxy mit ein bis neun Halogenatomen, Di$(C_1-C_4)$-Alkylamino, Cyano, Sulfo, Nitro,

A,X,Z unabhängig voneinander Sauerstoff, Schwefel, Sulfinyl oder Sulfonyl,

B Sauerstoff, Schwefel, Sulfinyl, Sulfonyl oder Stickstoff, der durch $(C_1-C_4)$-Alkyl substituiert sein kann; oder eine Doppelbindung,

C,D,E,F,G unabhängig voneinander Kohlenstoff oder Stickstoff,

V Kohlenstoff oder Phosphor, der durch $(C_1-C_6)$-Alkoxy, $(C_1-C_4)$-Alkylthio, Phenoxy oder Phenylthio, die beide ein bis drei Reste aus der Gruppe Halogen $(C_1-C_4)$-Alkyl, $(C_1-C_4)$-Alkoxy, Halo$(C_1-C_4)$-alkoxy, Cyano, Sulfo, Nitro tragen können, substituiert ist,

W $(C_1-C_{10})$-Alkylen, Halo$(C_1-C_{10})$-Alkylen, die durch ein bis fünf Reste der Gruppe $(C_1-C_4)$-Alkyl, Halo-$(C_1-C_4)$-alkyl, $(C_1-C_4)$-Alkoxy, Halo$(C_1-C_4)$-alkoxy, Cyano oder Nitro substituiert sein können,

$Y^1$ H, Halogen, $(C_1-C_4)$-Alkyl, Amino, $(C_1-C_4)$-Alkylamino, Di$(C_1-C_4)$-Alkylamino, Hydroxy,

$Y^2$ Sauerstoff, Schwefel oder Stickstoff, der durch $(C_1-C_4)$-Alkyl substituiert sein kann und

n,m unabhängig voneinander die Zahlen 0 bis 4 bedeuten, sowie deren in der Landwirtschaft einsetzbaren oder physiolgisch verträglichen Salze.

Besonders bevorzugte Verbindungen der Formeln I und Ia sind solche, worin

$R^1$, $R^2$ unabhängig voneinander, $(C_1-C_6)$-Alkyl, $(C_2-C_6)$-Alkenyl, $(C_2-C_6)$-Alkinyl, die durch ein bis dreizehn Halogenatome und/oder $(C_1-C_4)$-Alkoxy $(C_1-C_4)$-Alkoxycarbonyl, $(C_1-C_4)$-Alkylsulfenyl substituiert sein können,

$R^3$ Wasserstoff, $(C_1-C_6)$-Alkyl,

$R^4$ Wasserstoff, $(C_1-C_6)$-Alkyl, $(C_2-C_6)$-Alkenyl, $(C_2-C_6)$-Alkinyl, die durch ein- bis neun Halogenatome und/oder ein- bis dreifach durch einen Rest der Gruppe $(C_1-C_6)$-Alkoxy, Halo$(C_1-C_6)$-alkoxy, $(C_1-C_6)$-Alkoxycarbonyl, Halo$(C_1-C_6)$-alkoxycarbonyl, $(C_1-C_6)$-Alkylsulfenyl, $(C_1-C_6)$-Alkylsulfinyl, Di$(C_1-C_6)$-Alkylamino, Cyano, Sulfo, Nitro, Hydroxy, Carboxy substituiert sein können; $(C_1-C_6)$-Alkylsulfonyl, $(C_1-C_6)$-Alkylcarbonyl, Halo$(C_1-C_6)$-alkylsulfonyl, Halo$(C_1-C_6)$-alkylcarbonyl, Mono-, Di$(C_1-C_4)$-Alkylcarbamoyl,

$R^5$,$R^6$ unabhängig voneinander Halogen, $(C_1-C_4)$-Alkyl, $(C_1-C_4)$-Alkoxy, $(C_1-C_4)$-Alkylcarbonyl, $(C_1-C_4)$-Alkoxycarbonyl, $(C_1-C_4)$-Alkylsulfenyl, Halo$(C_1-C_4)$-alkoxy mit ein bis neun Halogenatomen, Di$(C_1-C_4)$-Alkylamino, Cyano, Sulfo, Nitro,

A,X,Z unabhängig voneinander Sauerstoff, Schwefel,

B Sauerstoff, Schwefel oder Stickstoff, der durch $(C_1-C_4)$-Alkyl substituiert sein kann; oder eine Doppelbindung

C,D,E,F,G unabhängig voneinander Kohlenstoff oder Stickstoff

V Kohlenstoff

W $(C_1-C_{10})$-Alkylen, Halo$(C_1-C_{10})$-Alkylen, die durch ein bis fünf Reste der Gruppe $(C_1-C_4)$-Alkyl, Halo-$(C_1-C_4)$-alkyl, $(C_1-C_4)$-Alkoxy oder Halo$(C_1-C_4)$-alkoxy substituiert sein können,

$Y^1$ H, Amino oder Hydroxy

$Y^2$ Sauerstoff, Schwefel oder Stickstoff, der durch $(C_1-C_4)$-Alkyl substituiert sein kann und

n,m unabhängig voneinander die Zahlen 0 bis 4 bedeuten, sowie deren in der Landwirtschaft einsetzbaren oder physiologisch verträglichen Salze.

Die Substituentenkette -A-W-B-$R^4$ steht bevorzugt in 2-, 3-. oder 4-Stellung am Phenylkern bzw. am N-haltigen Heterozyklus. In Abhängigkeit von der Position dieser Substituentenkette haben die verbleibenden Ringsubstituenten $R^5$ und $R^6$ insbesondere folgende Bedeutungen (Substitutionsmuster):

für 4-AWBR$^4$:

$R^5$: Wasserstoff, 2-Halogen, 2-$(C_1-C_6)$-Alkyl, 2-Carboxy, 2-$(C_1-C_6)$-Alkoxycarbonyl, 3-Halogen, 3-$(C_1-C_6)$-Alkyl, 3-Carboxy, 3-$(C_1-C_6)$-Alkoxycarbonyl

$R^6$: Wasserstoff, 5-Halogen;

für 3-AWBR$^4$:

$R^5$: Wasserstoff, 2-Halogen, 2-$(C_1-C_6)$-Alkyl, 2-Carboxy, 2-$(C_1-C_6)$-Alkoxycarbonyl

$R^6$: Wasserstoff, 4-Halogen, 5-Halogen, 6-Halogen oder

$R^5$: 4-Halogen, 4-$(C_1-C_6)$-Alkyl, 4-Carboxy, 4-$(C_1-C_6)$-Alkoxycarbonyl

$R^6$: Wasserstoff, 2-Halogen, 5-Halogen, 6-Halogen oder

$R^5$: 5-Halogen, 5-$(C_1-C_6)$-Alkyl, 5-Carboxy, 5-$(C_1-C_6)$-Alkoxycarbonyl,

$R^6$: Wasserstoff, 2-Halogen, 4-Halogen, 6-Halogen oder
$R^5$: 6-Halogen, 6-($C_1$-$C_6$)-Alkyl, 6-Carboxy, 6-($C_1$-$C_6$)-Alkoxycarbonyl
$R^6$: Wasserstoff, 2-Halogen, 4-Halogen, 5-Halogen;
für 2-AWBR$^4$:
$R^5$: Wasserstoff, 3-Halogen, 3-($C_1$-$C_6$)-Alkyl, 3-Carboxy, 3-($C_1$-$C_6$)-Alkoxycarbonyl,
$R^6$: Wasserstoff, 4-Halogen, 5-Halogen, 6-Halogen oder
$R^5$: 4-Halogen, 4-($C_1$-$C_6$)-Alkyl, 4-Carboxy, 4-($C_1$-$C_6$)-Alkoxycarbonyl
$R^6$: Wasserstoff, 3-Halogen, 5-Halogen, 6-Halogen oder
$R^5$: 5-Halogen,5-($C_1$-$C_6$)-Alkyl, 5-Carboxy, 5-($C_1$-$C_6$)-Alkoxycarbonyl,
$R^6$: Wasserstoff, 3-Halogen, 4-Halogen, 6-Halogen oder
$R^5$: 6-Halogen, 6-($C_1$-$C_6$)-Alkyl, 6-Carboxy, 6-($C_1$-$C_6$)-Alkoxycarbonyl
$R^6$: Wasserstoff, 3-Halogen, 4-Halogen, 5-Halogen.

Die Erfindung umfaßt auch alle Stereoisomeren und deren Gemische der Verbindungen der Formeln (I) und (Ia), wie die E- und Z- Isomeren bei ungesättigten Strukturen oder die optischen Isomeren falls Chiralitätszentren auftreten.

Als Salze der Verbindungen der Formeln I und Ia kommen insbesondere die Alkali-, Erdalkali-, Ammonium- oder ein-bis vierfach durch organische Reste (wie Alkyl, Hydroxyalkyl) substituierten Ammoniumsalze in Frage.

Die Salze werden insbesondere im Fall $R^4$ und/oder $Y^1$ = OH durch Deprotonierung des Sauerstoffs mit den entsprechenden Basen gebildet.

Als Basen für die Salzbildung werden bevorzugt die Hydroxide, Alkoholate, Hydride oder Metallalkyle oder -aryle der Alkali- bzw. Erdalkalimetalle wie beispielsweise Natrium- oder Kaliumhydroxid, Natriummethylat, -ethylat oder Kalium-tert.butylat, Natrium-, Kalium- oder Kalziumhydrid, Methyl-, n-Butyl-, tert. Butyl- oder Phenyllithium oder aber Ammoniak oder prim., sek. oder tert. Amine wie Methylamin, Diethylamin oder Triethylamin verwendet. Die eigentliche Umsetzung erfolgt nach für den Fachmann bekannten Methoden.

Gegenstand der vorliegenden Erfindung sind auch Verfahren zur Herstellung der Verbindungen (I) und (Ia), dadurch gekennzeichnet, daß man

a) eine Verbindung der Formel (II) oder (III)

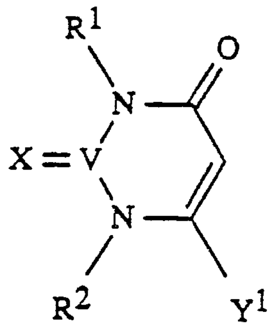

(II),

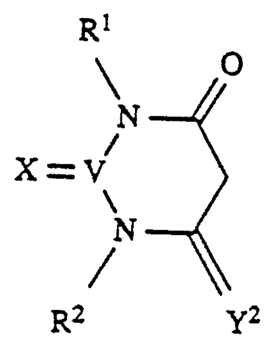

(III),

worin $R^1$,$R^2$,V,X,$Y^1$ und $Y^2$ dieselbe Bedeutung wie in den Formeln (I) und (Ia) besitzen, mit einer Verbindung der Formel (IV)

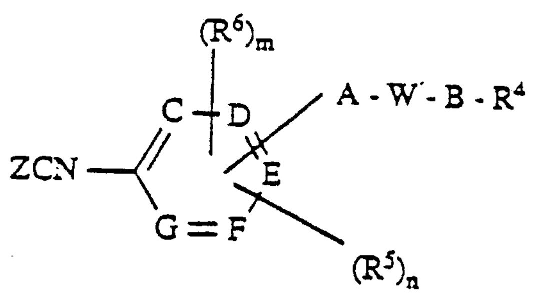

IV,

worin A B,C,D,E,F,G,W,Z,$R^4$,$R^5$,$R^6$,n und m dieselbe Bedeutung wie in Formeln (I) und (Ia) besitzen umsetzt oder

b) eine Verbindung der Formel (V) oder (VI),

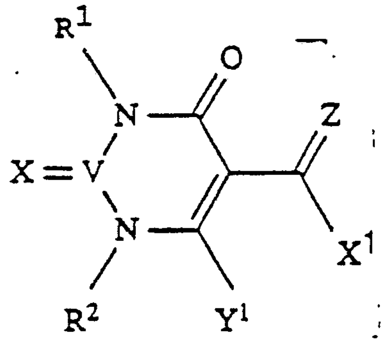

(V),

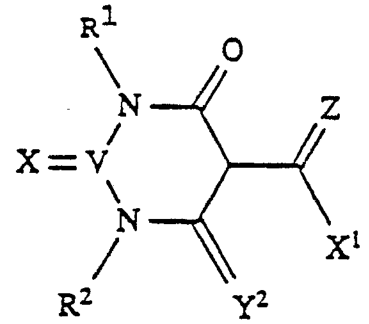

(VI),

worin $R^1$,$R^2$,V,X,$Y^1$,$Y^2$ und Z dieselbe Bedeutung wie in den Formeln (I) und (Ia) besitzen und X für eine nukleofuge Abgangsgruppe wie z.B. Halogen ($C_1$-$C_6$)-Alkoxy, ($C_1$-$C_6$)-Alkylthio, Phenyl, Imidazolyl oder Triazolyl steht, mit einer Verbindung der Formel (VII)

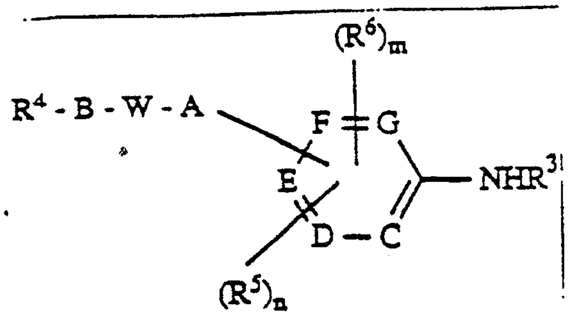

(VII),

worin A,B,C,D,E,F,G,W,$R^3$,$R^4$,$R^5$,$R^6$,n und m dieselbe Bedeutung wie in den Formeln (I) und (Ia) besitzen, umsetzt.

Die Herstellung der Pyrimidinderivate der Formeln (II) und (III) (z.B. Y=Hal, $NH_2$, OH, Beilstein E III/IV, Bd. 25, S.4108 - 4109 oder Beilstein II, Bd. 24, S. 270) und der Pyrimidin-5-carbonsäurederivate (V) und (VI) (z.B. Y=OH, $NH_2$, OEt-, OPh-Ester) sind literaturbekannt (K. Bredereck, R. Richter, Chem. Ber. 98, 131 (1965) bzw. J.-L.Bernier et al., Bull. Soc. Chim. France 1976, 616).

Die Verbindungen der Formel (IV) erhält man nach literaturbekannten Methoden aus den Aminen der Formel (VII) (Houben Weyl E4, S. 738 und S. 834).

Die Amine der Formel (VII) lassen sich nach literaturbekannten Verfahren herstellen, wie beispielsweise durch Umsetzung der entsprechenden Aminophenole mit fluorierten Olefinen (z.B. US 3409274) oder durch Reduktion der entsprechenden Nitroverbindungen (z.B. J. Soc. Dyers Col. 42, 78 (1926)).

Die Umsetzungen zur Darstellung der Verbindungen I bzw. Ia werden in Gegenwart eines gegenüber den Reaktionsteilnehmern inerten Lösungsmittels durchgeführt. In Betracht kommen vorzugsweise aliphatische und aromatische Kohlenwasserstoffe wie Cyclohexan, Toluol oder Xylol, Ether wie Dioxan, Glykoldimethylether, Tetrahydrofuran, chlorierte Kohlenwasserstoffe wie Methylenchlorid, Chloroform oder Tetrachlorkohlenstoff, Nitrile wie Acetonitril oder Gemische solcher Lösungsmittel.

Die Umsetzungen zur Darstellung der Verbindungen I oder Ia werden bei Temperaturen zwischen -10 °C und der Siedetemperatur des jeweils verwendeten Lösungsmittels und Reaktionszeiten von 10 Minuten bis 12 Stunden durchgeführt. Für $Y^1$ = Hydroxy werden Temperaturen von 50 °C - 120 °C und Reaktionszeiten von 1-4 Stunden bevorzugt, für $Y^1$ = Amino oder ($C_1$-$C_4$)-Alkylamino 80 - 180 °C und 2 - 10 Stunden. Die Reaktionen zur Salzbildung werden bevorzugt bei -10 - +70 °C und Reaktionszeiten von 10 Minuten - 5 Stunden durchgeführt.

Die Verbindungen der Formeln IV und VII sind neu und ebenfalls Gegenstand der vorliegenden Erfindung.

Die Wirkstoffe eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie

sind gegen normal sensible und resistente Arten sowie alle oder einzelne Entwicklungsstadien wirksam. Zu den oben erwähnten Schädlingen gehören:
Aus der Ordnung der Acarina z.B. Acarus siro, Agras spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.
Aus der Ordnung der Isopoda, z.B. Oniscus aselus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutiger 11a immaculata.
Aus der Ordung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung des Isoptera z.B. Reticulitermes spp. Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporarium, Aphis gossypii, Bravicornyne brassicae, Cryptomyzus ribis, Doralis fabae, Doralis pomi, Eriosoma lanigerum, Hyalopterus arundinis, Macrosiphium avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella auroantii, Apidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cocoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonumus spp., Sitophilus spp., Otiorrhynchus sulcatus, Cosmopolites sordidus, Ceuthorrhynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliophora erythrocephala, Lucilia spp., Chrysomyia spp., Cuterebra spp., Gastrophilus spp., Hypobosca spp., Stomoxys spp., Oestrus spp. Hypoderma spp., Tabanus spp., Tannia spp., Bibio hortulanus, Oscinella frit, Phorbia spp., Pegomyia hyoscyami, Ceratitis capitata, Dacus oleae, Tripula paludosa.
Aus der Ordnung der Siphonaptera z.B. Xenopsylla cheopsis, Ceratophyllus spp.
Aus der Ordnung der Arachnida z.B. Scorpio maurus, Latrodectus mactans.
Aus der Klasse der Helminthen z.B. Haemonchus, Trichostrongylus, Ostertagia; Cooperia, Chabertia, Strongyloides, Oesophagostomum, Hyostrongylus, Ancylostoma, Ascaris und Heterakis sowie Fasciola und pflanzenschädigende Nematoden z.B. solche der Gattungen Meloidogyne, Heterodera, Ditylenchus, Aphelenchoides, Radopholus, Globodera, Pratylenchus, Longidorus und Xiphinema.
Aus der Klasse der Gastropoda z.B. Deroceras spp., Arion spp., Lymnaea spp., Galba spp., Succinea spp., Biophalaria spp., Bulinus spp., Oncomelania spp.
Aus der Klasse der Bivalva z.B. Dreissena spp.

Die vorliegende Erfindung umfaßt somit auch Tierarzneimittel, dadurch gekennzeichnet, daß sie eine

wirksame Menge einer Verbindung der Formeln I oder Ia enthalten oder aus ihr bestehen.

Gegenstand der Erfindung sind auch Mittel, die die Verbindungen der Formel I neben geeigneten Formulierungshilfsmitteln enthalten.

Die erfindungsgemäßen Mittel enthalten die Wirkstoffe der Formel I oder Ia im allgemeinen zu 1 bis 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher infrage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in:

Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2′-dinaphthylmethan-6,6′-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr

Aktivsubstanz, vorzugsweise liegt sie jedoch zwischen 0,01 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a.

Bevorzugte Mischungspartner sind

1. aus der Gruppe der Phosphorverbindungen

Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulfphone, Dialifos, Diazinon, Dichlorvos, Dicrotophos, O,O-1,2,2,2-tetrachlorethylphosphorthioate (SD 208 304), Dimethoate, Disulfoton, EPN, Ethion, Ethoprophos, Etrimfos, Famphur, Fenamiphos, Fenitrothion, Fensulfothion, Fenthion, Fonofos, Formothion, Heptenophos, Isozophos, Isothioate, Isoxathion, Malathion, Methacrifos, Methamidophos, Methidation, Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;

2. aus der Gruppe der Carbamate

Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cumenylbutyryl (methyl)carbamate, Oxamyl, Pirimicarb, Propoxur, Thiodicarb, Thiofanox, Ethyl 4,6,9-triaza-4-benzyl-6,10-dimethyl-8-oxa-7-oxo-5,11-dithia-9-dodecenoate (OK 135), 1-methylthio(ethylideneamino)-N-methyl-N-(morpholinothio)carbamate (UC 51717);

3. aus der Gruppe der Carbonsäureester

Allethrin, Alphametrin, 5-Benzyl-3-furylmethyl-(E)-(1R)-cis-2,2-di-methyl-3-(2-oxothiolan-3-ylidenemethyl)-cyclopropanecarboxylate, Bioallethrin, Bioallethrin((S)-cyclopentylisomer), Bioresmethrin, Biphenate, (RS)-1-Cyano-1-(6-phenoxy-2-pyridyl)methyl-(1RS)-trans-3-(4-tert.butylphenyl)-2,2-dimethylcyclopropanecarboxylate (NCI 85193), Cycloprothrin, Cyfluthrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-isomer), Permethrin, Pheothrin ((R)-Isomer), d-Pralethrin, Pyrethrine (natürliche Produkte), Resmethrin, Tefluthrin, Tetramethrin, Tralomethrin;

4. aus der Gruppe der Amidine

Amitraz, Chlordimeform;

5. aus der Gruppe der Zinnverbindungen

Cyhexatin, Fenbutatinoxide;

6. Sonstige

Abamectin, Bacillus thuringiensis, Bensultap, Binapacryl, Bromopropylate, Buprofezin, Camphechlor, Cartap, Chlorobenzilate, Chlorfluazuron, 2-(4-(Chlorphenyl)-4,5-di-phenylthiophen (UBI-T 930), Chlorfentezine, Cyclopropancarbonsäure-(2-naphthylmethyl)ester (Ro 12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Dichlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl) (dimethyl)(3-(3-phenoxyphenyl)propyl)silan, (4-Ethoxyphenyl) (3-(4-fluoro-3-phenoxyphenyl)-propyl)dimethylsilan, Fenoxycarb, 2-Fluoro-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron.

Der Wirkstoffgehalt der aus den handelsüblichen Formulierungen bereiteten Anwendungsformen kann von 0,00000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich insbesondere auch zur Bekämpfung von Endo- und Ektoparasiten auf dem veterinärmedizinischen Gebiet bzw. auf dem Gebiet der Tierhaltung.

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Eine Pour on-Formulierung ist dadurch gekennzeichnet, daß der Wirkstoff in einem geeigneten hautverträglichen Lösungsmittel bzw. Lösungsmittelgemisch gegebenenfalls unter Zugabe weiterer Hilfsstoffe gelöst, emulgiert bzw. suspendiert wird und mit Hilfe einer geeigneten Vorrichtung (z.B. mit Hilfe eines Meßbechers, einer Sprühflasche oder einer Dosierspritze) auf die Haut des zu behandelnden Tieres gebracht wird.

In der Veterinärmedizin sind bereits Pour-on-Formulierungen von Isektiziden und Anthelmintika bekanntgeworden (s. dazu Rogoff, W.M. und Kohler, P.H., J. Econ. Ent. 53, 814-817 (1960) und EP-A 45242). Der Ausdruck "Pour-on-Formulierung" oder "Spot-on-Formulierung" ist dem Fachmann geläufig. Eine solche Formulierung stellt eine flüssige Präparation dar, die für die sogenannte "Pour-on-Applikation" geeignet ist und auf die Haut aufgegossen wird (Aufguß-Behandlung).

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern oder Schafen z.B. in Dosierungen von 0,01 mg bis 1 g/kg Körpergewicht eingesetzt werden.

Nachfolgende Beispiele dienen zur Erläuterung der Erfindung.

## A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

## B. Chemische Beispiele

a) Vorprodukte

3,5-Dichlor-4-(2-(1,1,2,2,3,3-hexafluorpropoxy)trifluorethoxy)-anilin

Zu 100,00 g (0.56 M) 3,5-Dichlor-4-aminophenol in 1100 ml wasserfreiem Acetonitril wurden 116,00 g (0,84 M) Kaliumcarbonat und anschließend 153,00 g (0,62 M) Trifluorvinyl-1,1,2,2,3,3-hexafluorpropylether gegeben und 2 Stunden bei 50 °C gerührt. Von Unlöslichem wurde abgesaugt, mit Methylenchlorid nachgewaschen und das Filtrat eingeengt. Der Rückstand wurde im Ölpumpenvakuum getrocknet.
Ausbeute: 203,40 g (85 % d. Th.) öliges Produkt

Analog dieser Vorschrift lassen sich auch die Verbindungen in Tab. 1 darstellen

Tabelle 1

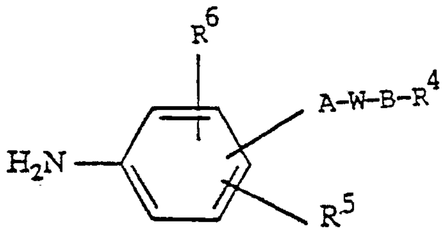

| Bsp. | $-A-W-B-R^4$ | $R^5$ | $R^6$ | Fp[°C] |
|---|---|---|---|---|
| a | $4-F_2CH(CF_2)_2O-CHFCF_2O$ | H | H | Schwarzes Öl |
| b | $4-F_2CH(CF_2)_2O-CHFCF_2O$ | 3-Cl | 5-Cl | " |
| c | $4-C_3F_7O-CHFCF_2O$ | 3-Cl | 5-Cl | " |
| d | $4-C_3F_7O-CF(CF_3)CF_2OCHFCF_2O$ | 3-Cl | 5-Cl | " |
| e | $2-F_2CH(CF_2)_2O-CHFCF_2O$ | H | H | Schwarzes ÖL |
| f | $2-F_2CH(CF_2)_2O-CHFCF_2O$ | 4-Cl | H | " |
| g | $2-F_2CH(CF_2)_2O-CHFCF_2O$ | 5-Cl | H | " |
| h | $3-F_2CH(CF_2)_2O-CHFCF_2O$ | H | H | " |
| i | 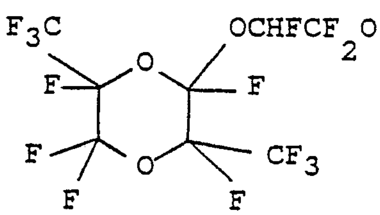 | 3-Cl | 5-Cl | " |
| j | $C_4F_9CF=CFCF_2O,$ | 3-Cl | 5-Cl | " |
| j1 | $2-F_2CH(CF_2)_2O-CHFCF_2O$ | $5-NO_2$ | H | " |
| j2 | $2-F_2CH(CF_2)_2O-CHFCF_2O$ | $4-CH_3$ | H | " |
| j3 | $2-F_2CH(CF_2)_2O-CHFCF_2O$ | $5-CH_3$ | H | " |
| j4 | $3-F_2CH(CF_2)_2O-CHFCF_2O$ | 4-Cl | H | " |
| j5 | $3-F_2CH(CF_2)_2O-CHFCF_2O$ | 6-Cl | H | " |

| Bsp. | -A-W-B-$R^4$ | $R^5$ | $R^6$ | $n_D(T[°C])$ |
|---|---|---|---|---|
| j6 | 4-$F_2CH(CF_2)_2O$-$CHFCF_2O$ | 2-$CH_3$ | H | 1,4150 (22,5) |
| j7 | 2- " | 4-$CH_3$, | 5-Cl | 1,4120 (23) |
| j8 | 4- " | 2,6-$(CH_3)_2$ | | 1,4213 (21) |
| j9 | 2- " | 5-$CF_3$ | H | 1,3895 (21) |
| j10 | 2- " | 3,5,6-$Cl_3$ | | 1,4372 (22) |
| j11 | 2- " | 3-$CH_3$ | H | Öl |
| J12 | 4- " | 2,3-$(CH_3)_2$ | | Öl |
| J13 | 3- " | 2,5-$(CH_3)_2$ | | Öl |
| J14 | 2- " | 3,5-$(CH_3)_2$ | | 1,4483 (23) |
| J15 | 3- " | 2-$CH_3$ | H | 1,4045 (23) |
| J16 | 2-$CF_3$-$CFBrCF_2O$-$CHFCF_2O$ | 4-Cl | H | Öl |
| J17 | 2- " | 4-$CH_3$ | H | Öl |
| J18 | 4-$F_2CBrCF_2O$-$CHFCF_2O$ | 3,5-$Cl_2$ | | Öl |
| J19 | 2- " | 4-Cl | H | Öl |
| J20 | 2- " | 4-$CH_3$ | H | Öl |
| J21 | 2- " | 5-$CH_3$ | H | ÖL |
| J22 | 4- " | 2,6-$(CH_3)_2$ | | 1,4515 (26) |
| J23 | 2- " | 5-$CF_3$ | H | 1,4122 (26) |
| J24 | 2- " | 3,5,6-$Cl_3$ | | 1,4658 (26) |
| J25 | 4- " | 2-$CH_3$ | H | 1,4380 (22) |
| J26 | 2- " | 4-$CH_3$ | 5-Cl | 1,4653 (26) |
| J27 | 2- " | 5-Cl | H | Öl |
| J28 | 4-$F_2CH(CF_2)_4O$-$CHFCF_2O$ | 3,5-$Cl_2$ | | Öl |
| J29 | 2- " | 4-$CH_3$ | H | 1,3900 |
| J30 | 2- " | 4-Cl | H | Öl |
| J31 | 2- " | 5-Cl | H | Öl |
| J32 | 2- " | 5-$CH_3$ | H | 1,3947 (22) |
| J33 | 4- " | 2-$CH_3$ | H | 1,3945 (24) |
| J34 | 2- " | 4-$CH_3$ | 5-Cl | 1,4265 (28) |
| J35 | 2- " | 3,5,6-$Cl_3$ | | 1,4143 (26) |
| J36 | 4- " | H | H | Öl |
| J37 | 2- " | 5-$CF_3$ | H | 1,3723 (30) |
| J38 | 4-$CF_2$=CFO-$(CF_2)_6$-O-$CHFCF_2O$ | 3,5-$Cl_2$ | | Öl |
| J39 | 2- " | 5-$CF_3$ | H | 1,3962 (26) |

| Bsp. | -A-W-B-$R^4$ | $R^5$ | $R^6$ | $n_D(T[°C])$ |
|---|---|---|---|---|
| J40 | 4-$F_3C(CF_2)_2O$-$CHFCF_2O$ | 3,5-$Cl_2$ | | ÖL |
| J41 | 2-$F_2CH(CF_2)_2O$-$CHFCF_2S$ | H | H | 1,4501 (26,5) |
| J42 | 4- " | H | H | 1,4590 (24,5) |
| J43 | 3- " | H | H | 1,4441 (26) |
| J44 | 4- " | 2-F | H | 1,4420 (19) |
| J45 | 4- " | 2,5-$CH_3$ | | 1,4590 (22) |
| J46 | 4- " | 2-Br | H | 1,4966 (23,5) |
| J47 | 4- " | 2-$CH_3O$ | H | 1,4825 (30) |
| J48 | 4- " | 2-$CH_3$ | H | 1,4628 (22) |
| J49 | 4- " | 2,5-$Cl_2$ | | 1,4721 (23) |
| J50 | 4- " | 2-F | 3-Cl | Öl |
| J51 | 4- " | 2-CN | H | Öl |
| J52 | 2-$BrCF_2CF_2$-O-CHF-$CF_2S$ | H | H | Öl |
| J53 | 4- " | H | H | Öl |
| J54 | 3- " | H | H | Öl |
| J55 | 4- " | 2,5-$(CH_3)_2$ | | 1,4404 (26) |
| J56 | 4- " | 2-F | H | 1,4733 (29) |
| J57 | 4- " | 2-Br | H | 1,5294 (26,5) |
| J58 | 4- " | 2,5-$Cl_2$ | | 1,4989 (28) |
| J59 | 3-$F_2CH$-$(CF_2)_4OCHF$-$CF_2S$ | H | H | 1,4257 (26) |
| J50 | 2- " | H | H | 1,4320 (20) |
| J51 | 4- " | H | H | Öl |
| J52 | 2-$CF_2$=$CFO(CF_2)_6OCHFCF_2S$ | H | H | Öl |
| J53 | 4- " | H | H | Öl |

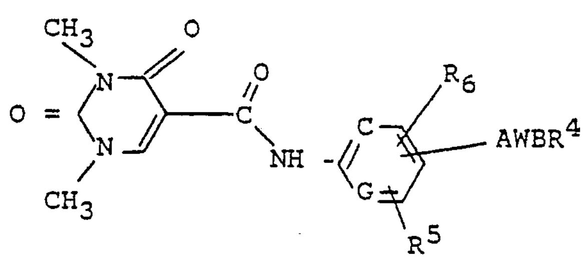

| Bsp. | -A-W-B-$R^4$ | $R^5$ | $R^6$ | C | G | $n_D(T[°C])$ |
|---|---|---|---|---|---|---|
| J54 | 3-$F_2CH(CF_2)_2O$-$CHFCF_2O$ | 5-$CH_3$ | H | N | N | Öl |
| J55 | 2- " | H | H | N | CH | Öl |

3,5-Dichlor-4-(2-(1,1,2,2,3,3-hexafluorpropoxy)-trifluorethoxy)-phenylisocyanat

42,60 g (0,10 M) 3,5-Dichlor-4-(2-(1,1,2,2,3,3-hexafluorpropoxy)-trifluorethoxy)-anilin und 50 g Phosgen in 250 ml absoluten Toluol wurden langsam von 0 °C auf Rückflußtemperatur erhitzt. Das überschüssige Phosgen wurde mit Stickstoff vertrieben und das Lösungsmittel eingeengt. Das erhaltene Öl wurde ohne weitere Reinigung eingesetzt.
Ausbeute: 38,4 g (85 % d. Th.)

Tabelle 2

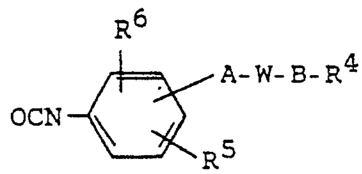

Analog zu dieser Vorschrift lassen sich die Verbindungen k-t herstellen.

| Bsp. | $-A-W-B-R^4$ | $R^5$ | $R^6$ | Fp[°C] |
|---|---|---|---|---|
| k | $4-F_2CH(CF_2)_2O-CHFCF_2O$ | H | H | |
| l | $4-F_2CH(CF_2)_2O-CHFCF_2O$ | 3-Cl | 5-Cl | |
| m | $4-C_3F_7O-CHFCF_2O$ | 3-Cl | 5-Cl | |
| n | $4-C_3F_7O-CF(CF_3)CF_2OCHFCF_2O$ | 3-Cl | 5-Cl | |
| o | $2-F_2CH(CF_2)_2O-CHFCF_2O$ | H | H | |
| p | $2-F_2CH(CF_2)_2O-CHFCF_2O$ | 4-Cl | H | |
| q | $2-F_2CH(CF_2)_2O-CHFCF_2O$ | 5-Cl | H | |
| r | $3-F_2CH(CF_2)_2O-CHFCF_2O$ | H | H | |
| s | 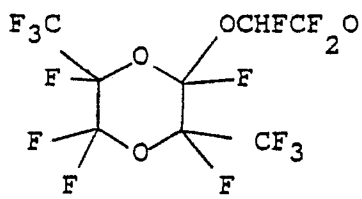 | 3-Cl | 5-Cl | |
| t | $C_4F_9CF=CFCF_2O$, | 3-Cl | 5-Cl | |

b) Endprodukte

1,3-Dimethyl-5-(4-(2-(1,1,2,2,3,3-hexafluorpropoxy)-1,1,2-trifluorethoxy)-phenylcarbamoyl)-barbitursäure (Bsp. 1)

9,26 g (40,6 mM) 1,3-Dimethyl-5-ethoxycarbonylbarbitursäure in 100 ml Toluol wurden mit 14,50 g (40,6 mM) 4-(2-(1,1,2,2,3,3,-hexafluorpropoxy)-1,1,2-trifluorethoxy)anilin versetzt und 2,5 Stunden unter Rückfluß gerührt. nach Einengen wurde aus Methanol umkristallisiert.
Ausbeute: 9,50 (48 % d. Th.)

Fp.: 126 °C

1,3-Dimethyl-5-(3,5-dichlor-(4-(2-(1,1,2,2,3,3-hexafluorpropoxy)-1,1,2-trifluorethoxy))-phenylcarbamoyl)-barbitursäure (Bsp. 4)

3,20 g (20,5 mM) 1,3-Dimethylbarbitursäure in 40 ml Toluol wurden bei Raumtemepratur mit 1,04 g (10,3 mM) Triethylamin und anschließend mit 9,25 g (20,5 mM) 3,5-Dichlor-(4-(2-(1,1,2,2,3,3-hexafluorpropoxy)-1,1,2-trifluorethoxy))-phenylisocyanat versetzt. Bei 40 °C wurde 2 Stunden nachgerührt, mit 200 ml Methylenchlorid versetzt und mit 100 ml 2n wässriger Salzsäure und mehrfach mit Wasser gewaschen. Nach Trocknen über Magnesiumsulfat wurde eingeengt.
Ausbeute: 11,30 (90 % d. Th.)
Fp.: 137 °C

1,3-Dimethyl-5-(3,5-dichlor-(4-(2-(1,1,2,2,3,3,3-heptafluorpropoxy)-1,1,2-trifluorethoxy))-phenylcarbomamoyl)-barbitursäure, Triethylammoniumsalz (Bsp. 15)

2,25 g (3,34 mM) 1,3-Dimethyl-5-(3,5-dichlor-(4-(2-(1,1,2,2,3,3,3-heptafluorpropoxy)-1,1,2-trifluorethoxy))-phenylcarbamoyl) barbitursäure in 30 ml Acetonitril wurden bei Raumtemperatur mit 0,38 g (3,7 mM) Triethylamin 2 Stunden gerührt. Die Lösung wurde eingeengt und der Rückstand bei 50 °C im Ölpumpenvakuum getrocknet.
Ausbeute: 2,90 g (98 % d. Th.) harziges Produkt

1,3-Dimethyl-5-(3,5-dichlor-(4-(2-(1,1,2,2,3,3-hexafluorpropoxy)-1,1,2-trifluorethoxy))-phenylcarbamoyl)-barbitursäure, Ammoniumsalz (Bsp. 5)

5,0 g (8,2 mM) 1,3-Dimethyl-5-(3,5-dichlor-(4-(2-(1,1,2,2, 3,3-hexafluorpropoxy)-1,1,2-trifluorethoxy))-phenylcarbamoyl)barbitursäure wurden in 50 ml abs. Acetonitril suspendiert. Es wurde 10 Minuten lang bei Raumtemperatur Ammoniak eingeleitet und 10 Minuten bei 50 °C gerührt. Der ausgefallene Niederschlag wurde abgesaugt, mit Ether gewaschen und getrocknet.
Ausbeute: 4,4 g (86 % d. Th.)
Fp.: 133 - 161 °C

Analog zu diesen Vorschriften lassen sich die Verbindungen der Formel I der nachfolgenden Tabelle 3 darstellen.

**Tabelle 3**

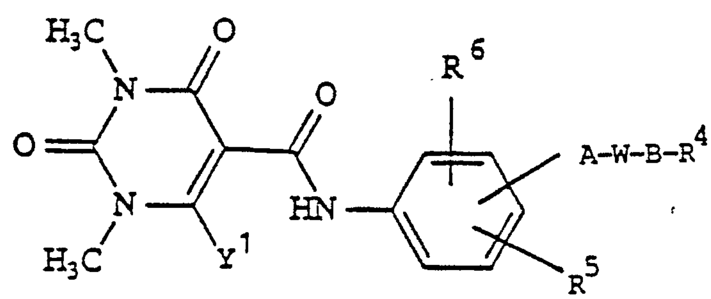

(I)

| Bsp. | $-A-W-B-R^4$, $R^5$, $R^6$ | $Y^1$ | Fp[°C] |
|---|---|---|---|
| 1 | $4-F_2CH(CF_2)_2O-CHFCF_2O,H,H$ | OH | 126 |
| 2 | $4-F_2CH(CF_2)_2O-CHFCF_2O,H,H$ | $O^-NH_4^+$ | 145 |
| 3 | $4-F_2CH(CF_2)_2O-CHFCF_2O,H,H$ | $O^-NH(C_2H_5)_3^+$ | 134 |
| 4 | $4-F_2CH(CF_2)_2O-CHFCF_2O,3,5-Cl_2$ | OH | 139 |
| 5 | $4-F_2CH(CF_2)_2O-CHFCF_2O,3,5-Cl_2$ | $O^-NH_4+$ | 133-161 |
| 6 | $4-F_2CH(CF_2)_2O-CHFCF_2O,3,5-Cl_2$ | $O^-NH(C_2H_5)_3^+$ | Harz |
| 7 | $4-F_2CH(CF_2)_2O-CHFCF_2O,3,5-Cl_2$ | $O^-Li^+$ | 136-139 |
| 8 | $4-F_2CH(CF_2)_2O-CHFCF_2O,3,5-Cl_2$ | $O^-Na^+$ | >280 |
| 9 | $4-F_2CH(CF_2)_2O-CHFCF_2O,3,5-Cl_2$ | $O^-K^+$ | >270 |
| 10 | $4-C_3F_7O-CHFCF_2O,3,5-Cl_2$ | OH | 145 |
| 11 | $4-C_3F_7O-CHFCF_2O,3,5-Cl_2$ | $O^-NH_4+$ | 160-162 |
| 12 | $4-C_3F_7O-CHFCF_2O,3,5-Cl_2$ | $O^-NH(C_2H_5)_3^+$ | Harz |
| 13 | $4-C_3F_7O-CF(CF_3)CF_2OCHFCF_2O,3,5-Cl_2$ | OH | 124 |
| 14 | $4-C_3F_7O-CF(CF_3)CF_2OCHFCF_2O,3,5-Cl_2$ | $O^-NH_4^+$ | 148 |
| 15 | $4-C_3F_7O-CF(CF_3)CF_2OCHFCF_2O,3,5-Cl_2$ | $O^-NH(C_2H_5)_3)^+$ | Harz |
| 16 | $2-F_2CH(CF_2)_2O-CHFCF_2O,H,H$ | OH | 144 |
| 17 | $2-F_2CH(CF_2)_2O-CHFCF_2O,H,H$ | $O^-NH(C_2H_5)_3^+$ | 93-94 |
| 18 | $2-F_2CH(CF_2)_2O-CHFCF_2O,H,H$ | $O^-K^+$ | >270 |
| 19 | $2-F_2CH(CF_2)_2O-CHFCF_2O,4-Cl,H$ | OH | 176,5 |
| 20 | $2-F_2CH(CF_2)_2O-CHFCF_2O,4-Cl,H$ | $O^-NH(C_2H_5)_3^+$ | |
| 21 | $2-F_2CH(CF_2)_2O-CHFCF_2O-4-Cl,H$ | $O^-K^+$ | |
| 22 | $2-F_2CH(CF_2)_2O-CHFCF_2O,5-Cl,H$ | OH | 121 |
| 23 | $2-F_2CH(CF_2)_2O-CHFCF_2O,5-Cl,H$ | $O^-NH(C_2H_5)_3^+$ | |
| 24 | $2-F_2CH(CF_2)_2O-CHFCF_2O,5-Cl,H$ | $O^-K^+$ | |
| 25 | $3-F_2CH(CF_2)_2O-CHFCF_2O,H,H$ | OH | 97 |
| 26 | $3-F_2CH(CF_2)_2O-CHFCF_2O,H,H$ | $O^-NH(C_2H_5)_3^+$ | Öl |
| 27 | $3-F_2CH(CF_2)_2O-CHFCF_2O,H,H$ | $O^-K^+$ | >270 |

Fortsetzung Tabelle 3

| Bsp. | $-A-W-B-R^4$, $R^5$, $R^6$ | $Y^1$ | Fp[°C] |
|---|---|---|---|
| 28 | 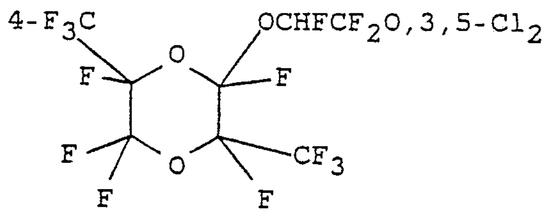 | OH | 135 |
| 29 | 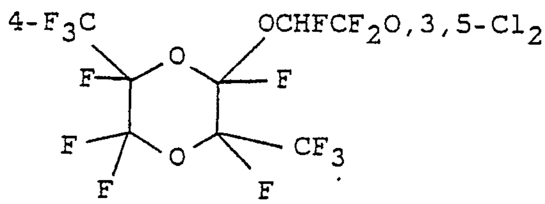 | $O^-NH(C_2H_5)_3{}^+$ | Harz |
| 30 | $4-C_4F_9CF=CFCF_2O,3,5-Cl_2$ | OH | 114 |
| 31 | $4-F_2CH(CF_2)_2O-CHFCF_2O,3,5-Cl_2$ | $NH_2$ | 174 |
| 32 | $3-F_2CH(CF_2)_2O-CHFCF_2O,4-Cl,H$ | OH | 92 |
| 33 | $3-F_2CH(CF_2)_2O-CHFCF_2O,4-Cl,H$ | $O^-N(C_2H_5)_3{}^+$ | Öl |
| 34 | " | $O^-K^+$ | |
| 35 | $3-F_2CH(CF_2)_2O-CHFCF_2O,6-Cl,H$ | OH | 99 |
| 36 | " | $O^-N(C_2H_5)_3{}^+$ | Öl |
| 37 | " | $O^-K^+$ | |
| 38 | $2-F_2CH(CF_2)_2O-CHFCF_2O,5-NO_2,H$ | OH | 172 |
| 39 | " | $O^-N(C_2H_5)_3{}^+$ | |
| 40 | " | $O^-K^+$ | |
| 41 | $2-F_2CH(CF_2)_2O-CHFCF_2O,4-CH_3,H$ | OH | 156 |
| 42 | " | $O^-N(C_2H_5)_3{}^+$ | 128-145 |
| 43 | " | $O^-Na^+$ | >280 |
| 44 | " | $O^-K^+$ | >280 |
| 45 | $2-F_2CH(CF_2)_2O-CHFCF_2O,5-CH_3,H$ | OH | 152 |
| 46 | " | $O^-N(C_2H_5)^+$ | Harz 116-200 |

Fortsetzung Tabelle 3

| Bsp. | -A-W-B-$R^4$, $R^5$, $R^6$ | $Y^1$ | Fp[°C] |
|---|---|---|---|
| 47 | " | $O^-Na^+$ | >280 |
| 48 | " | $O^-K^+$ | >280 |
| 49 | 4-$CH_3O(CH_2)_2O$,3,5-$Cl_2$ | OH | 168 |
| 50 | " | $O^-N(C_2H_5)_3{}^+$ | |
| 51 | 4-$CH_3O(CH_2)_2O$,H,H | OH | 170 |
| 52 | 4-$C_2H_5O(CH_2)_2O$,3,5-$Cl_2$ | OH | 154 |
| 53 | 4-$C_2H_5O(CH_2)_2O$,H,H | OH | 195-208 |
| 54 | 3-$CH_3O(CH_2)_2O$,H,H | OH | 140 |
| 55 | 3-$C_2H_5O(CH_2)_2O$,H,H | OH | 133 |
| 56 | 3-$CH_3O(CH_2)_2O$,2-$CH_3$,H | OH | |
| 57 | 3-$C_2H_5O(CH_2)_2O$,2-$CH_3$, H | OH | |
| 58 | 2-$CH_3O(CH_2)_2O$,H,H | OH | 138 |
| 59 | 2-$C_2H_5O(CH_2)_2O$,H,H | OH | 135 |
| 60 | 2-$CH_3O(CH_2)_2O$,5-Br,H | OH | 190 |
| 61 | 2-$C_2H_5O(CH_2)_2O$,5-Br,H | OH | 175 |
| 62 | 2-$CH_3O(CH_2)_2O$,3,5-$Br_2$ | OH | 179 |
| 63 | 2-$CH_3O(CH_2)_2O$,6-$CH_3$,H | OH | |
| 64 | 2-$C_2H_5O(CH_2)_2O$,6-$CH_3$,H | OH | |
| 65 | 4-$CH_3O(CH_2)_2O$,3-Cl,H | OH | 146 |
| 66 | 4-$C_2H_5O(CH_2)_2O$,3-Cl,H | OH | 126 |
| 67 | 2-$CH_3O(CH_2)_2O$,3-Cl,H | OH | 150 |
| 68 | 2-$C_2H_5O(CH_2)_2O$,3-Cl,H | OH | 118 |

Fortsetzung Tabelle 3

| Bsp. | -A-W-B-$R^4$, $R^5$, $R^6$ | $Y^1$ | Fp[°C] |
|---|---|---|---|
| 69 | 4-$F_2CH(CF_2)_2O$-$CHFCF_2O$,2-$CH_3$,H | OH | 135 |
| 70 | 2- " 4-$CH_3$,5-Cl | OH | 137 |
| 71 | 4- " 2,6-($CH_3$) | OH | 139 |
| 72 | 2- " 5-$CF_3$,H | OH | 156 |
| 73 | 2- " 3,5,6-$Cl_3$ | OH | 120 |
| 74 | 2- " 3-$CH_3$,H | OH | 137 |
| 75 | 4- " 2,3-$(CH_3)_2$ | OH | 130 |
| 76 | 4- " 2,5-$(CH_3)_2$ | OH | 129 |
| 77 | 2- " 3,5-$(CH_3)_2$ | OH | 150 |
| 78 | 2-$CF_3$-CFBr-$CF_2O$-$CHFCF_2O$,4-Cl,H | OH | 115 |
| 79 | 2- " 4-$CH_3$,H | OH | 137 |
| 80 | 4-$F_2CBrCF_2O$-$CHFCF_2O$,3,5-$Cl_2$ | OH | 127 |
| 81 | 2- " 4-Cl,H | OH | 141 |
| 82 | 2- " 4-$CH_3$,H | OH | 139 |
| 83 | 2- " 5-$CH_3$,H | OH | 146 |
| 84 | 4- " 2,6-$(CH_3)_2$ | OH | 113 |
| 85 | 2- " 5-$CF_3$, H | OH | 157 |
| 86 | 2- " 3,5,6-$Cl_3$ | OH | 146 |
| 87 | 4- " 2-$CH_3$, H | OH | 118 |
| 88 | 2- " 4-$CH_3$, 5-Cl | OH | 159 |
| 89 | 2- " 5-Cl, H | OH | 172 |
| 90 | 4-$F_2CH(CF_2)_4O$-$CHFCF_2O$,3,5-$Cl_2$ | OH | 126 |
| 91 | 2- " 4-$CH_3$,H | OH | 130 |
| 92 | 2- " 4-Cl,H | OH | 102 |
| 93 | 2- " 5-Cl,H | OH | 162 |
| 94 | 2- " 5-$CH_3$,H | OH | 139 |
| 95 | 4- " 2-$CH_3$,H | OH | 110 |
| 96 | 2- " 4-$CH_3$,5-Cl | OH | 114 |
| 97 | 2- " 3,5,6-$Cl_3$ | OH | 138 |
| 98 | 4- " H,H | OH | 93 |
| 99 | 2- " 5-$CF_3$,H | OH | 138 |
| 100 | 4-$CF_2$=CFO-$(CF_2)_6$-O-$CHFCF_2O$,3,5-$Cl_2$ | OH | 192 |
| 101 | 2- " 5-$CF_3$,H | OH | 215 |

Fortsetzung Tabelle 3

| Bsp. | $-A-W-B-R^4$, $R^5$, $R^6$ | $Y^1$ | Fp[°C] |
|---|---|---|---|
| 102 | 4-$F_3C(CF_2)_2O-CHFCF_2O$,3,5-$Cl_2$ | $NH_2$ | 192 |
| 103 | 2-$F_2CH(CF_2)_2O-CHFCF_2S$,H,H | OH | 121 |
| 104 | 4- " ,H,H | OH | 119 |
| 105 | 3- " ,H,H | OH | 102 |
| 106 | 4- " ,2-F,H | OH | 114 |
| 107 | 4- " ,2,5-$(CH_3)_2$ | OH | 132 |
| 108 | 4- " ,2-Br,H | OH | 96 |
| 109 | 4- " ,2-$CH_3O$,H | OH | 144 |
| 110 | 4- " ,2-$CH_3$,H | OH | 111 |
| 111 | 4- " ,2,5-$Cl_2$ | OH | 145 |
| 112 | 4- " ,2-F,3-Cl | OH | 114 |
| 113 | 4- " ,2-CN,H | OH | 105 |
| 114 | 2-$BrCF_2CF_2O-CHF-CF_2S$,H,H | OH | 85-87 |
| 115 | 4- " ,H,H | OH | 102-104 |
| 116 | 3- " ,H,H | OH | 89 |
| 117 | 4- " ,2,5-$(CH_3)_2$ | OH | 132 |
| 118 | 4- " ,2-F,H | OH | 107 |
| 119 | 4- " ,2-Br,H | OH | 124 |
| 120 | 4- " ,2,5-$Cl_2$ | OH | 132 |
| 121 | 3-$F_2CH-(CF_2)_4OCHF-CF_2S$,H,H | OH | 102 |
| 122 | 2- " ,H,H | OH | 70 |
| 123 | 4- " ,H,H | OH | 84 |
| 124 | 2-$CF_2=CFO(CF_2)_6OCHFCF_2S$,H,H | OH | 140 |
| 125 | 4- " ,H,H | OH | 168 |
| 126 | 3-$C_2H_5O(CH_2)_2O$,2-$CH_3$,H | OH | Harz |
| 127 | 2- " ,3,5-$Br_2$ | OH | 158 |
| 128 | 4-$C_4H_9O(CH_2)_2O$,H,H | OH | 119 |
| 129 | 2- " ,3,5-$Br_2$ | OH | 137 |
| 130 | 4- " ,3,5-$Cl_2$ | OH | 132 |
| 131 | 4-$CH_3O-(CH_2)_2O-(CH_2)_2O$,H,H | OH | 134-135 |
| 132 | 4- " ,3,5-$Cl_2$ | OH | 135 |
| 133 | 2- " ,3,5-$Br_2$ | OH | 132 |

Fortsetzung Tabelle 3

| Bsp. | $-A-W-B-R^4$, $R^5$, $R^6$ | $Y^1$ | Fp[°C] |
|---|---|---|---|
| 134 | $4-CH_3O-CH(CH_3)(CH_2)_2O,H,H$ | OH | 115 |
| 135 | 4- " $,3,5-Cl_2$ | OH | 176 |
| 136 | $4-CH_3O-CH_2CH(CH_3)O,H,H$ | OH | 110-112 |
| 137 | 4- " $,3,5-Cl_2$ | OH | 178 |
| 138 | 2- " $,3,5-Br_2$ | OH | 169 |
| 139 | $4-CH_3O(CH_2)_2O-(CH_2)_2S,H,H$ | OH | 85 |
| 140 | 4- " ,2-Br,H | OH | |
| 141 | $4-CH_3O(CH_2)_2S,H,H$ | OH | 96 |
| 142 | $4-C_2H_5O(CH_2)_2S,H,H$ | OH | 86 |
| 143 | $4-C_4H_9O(CH_2)_2S,H,H$ | OH | 86 |
| 144 | $4-CH_3O-CH(CH_3)CH_2S,H,H$ | OH | 91 |

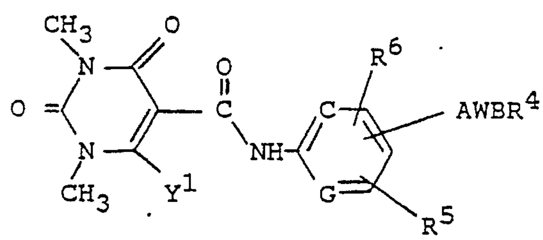

| Bsp. | $-A-W-B-R^4$, $R^5$, $R^6$ | C | G | $Y^1$ | Fp[°C] |
|---|---|---|---|---|---|
| 145 | $3-F_2CH(CF_2)_2O-CHFCF_2O,H,H$ | N | C | OH | 142 |
| 146 | $3-F_2CH(CF_2)_2O-CHFCF_2O,5-CH_3,H$ | N | N | OH | |
| 147 | $3-CH_3O(CH_2)_2O,H,H$ | N | C | OH | |
| 148 | $3-C_2H_5O(CH_2)_2O,H,H$ | N | C | OH | |
| 149 | $3-CH_3O(CH_2)_2O,5-CH_3,H$ | N | N | OH | |
| 150 | $3-C_2H_5O(CH_2)_2O,5-CH_3,H$ | N | N | OH | |
| 151 | $3-F_2CH(CF_2)_2O-CHFCF_2O,5-CH_3,H$ | N | N | OH | 175-202 |
| 152 | 2- " ,H,H | N | CH | OH | 142 |
| 153 | 2- " ,H,H | N | CH | $O^-NH(C_2H_5)_3^+$ | Harz |

## C. Biologische Beispiele:

**Beispiel 1** (Lucilia-Test):

Die Substanzen werden in einer Mischung bestehend aus Dimethylformamid (85 g), ®Emulsogen (7 g) und ®Arkopal N 60 (3 g) so gelöst, daß Verdünnungsreihen mit Wirkstoffkonzentrationen von 10000/1000

ppm erhalten werden. Jeweils 1 ml dieser Lösungen wird mit 9 g fein vermahlenem Fleisch innig vermischt, so daß letztlich Fleisch mit Wirkstoffkonzentrationen von 1000/100 ppm vorhanden ist. Zur Kontrolle werden 9 g Fleisch mit 1 ml des Lösungsmittels versetzt.

Den so präparierten Larvennährmedien werden jeweils 20 frisch geschlüpfte Larven von Lucilia cuprina zugegeben. Nach 72 Stunden, wenn sich im Kontrollmedium die Larven I zu den verpuppungsreifen Larven III entwickelt haben, wird die Mortalitätsrate ermittelt. In diesem Test erbrachten die Verbindungen gemäß Beispielen 3, 4, 6, 10, 12, 28 und 29 (Tab. 3) bei einer Wirkstoffkonzentration von 100 ppm 100% Abtötung der Larven.

**Beispiel** 2 (Nematoden-Test; in vitro)

Bei in vitro-Versuchen werden Nematoden in einem Kulturmedium gehalten, dem bis zu 5 Tagen die Testsubstanz zugegeben wird. Im Vergleich zu unbehandelten Kontrollkulturen wird die Motilität der Nematoden und deren Reproduktion am 6. Versuchstag untersucht und bemessen.

**Beispiel** 3 (Nematoden-Test; in vivo):

Bei Nematoden-Versuchen werden Labor-Nager, Hunde (± 10 kg KM) oder Lämmer (± 20 kg KM) mit deren natürlichen oder adaptierten Nematoden infiziert, nach Ablauf der Präpatenzperiode wird koproskopisch mehrfach die Ei-Ausscheidung bis zur Stabilisierung kontrolliert. Anschließend erfolgt die Behandlung mit der Testsubstanz, ein- oder mehrfach, in verschiedenen Applikationsrouten und bei unterschiedlichen Dosierungen. Maximal werden beim Labor-Nager 400 mg/kg KM, beim Großtier 50 mg/kg KM gegeben. Kontrolliert wird das Ergebnis der Behandlung koproskopisch bis zu vier Wochen p.appl., bei Reduktion der Ei-Ausscheidung um mehr als 90 % erfolgt helminthologische Sektion und Vergleich der Wurmbürde zu einem nicht behandelten Kontroll-Kollektiv.

**Beispiel** 4 (Nematoden-Test):

Lämmer im Gewicht von ± 20 kg KM werden oral mit Metazerkarien des Trematoden Fasciola hepatica infiziert. Nach Ablauf der Präpatenzperiode wird wiederholt koproskopisch die Höhe der Ausscheidung von Fasciola-Eiern überprüft; nach Stabilisierung der Ei-Ausscheidung erfolgt die medikamentöse Behandlung mit von Fall zu Fall wechselndem Applikationsweg. Die Dosierung ist gleichfalls variabel, ein- oder mehrfach gegeben, maximal beträgt sie 50 mg/kg KM. Die Erfolgskontrolle wird koproskopisch bis zu vier Wochen p.appl. vorgenommen, bei Ei-Reduktion um mehr als 90 % folgt helminthologische Sektion. Die Auswertung geschieht als 'controlled test', d.h. im Vergleich zu einem unbehandelten Versuchs-Kollektiv.

| Bsp. gemäß Tab. 3 | Anthelminthische Wirksamkeit Fasciola hepatica | gastro-intestinale Nematoden | in vitro-Screening Helminthen Motilität/Reproduktion | |
|---|---|---|---|---|
| | 1x10 bis 50 mg/kg KM p.o. | 1x10 bis 50 mg/kg KM p.o. und/oder S.c. | | |
| 4 | + + + | + + + | + + + | + + + |
| 10 | + + + | + + + | + + + | + + + |
| 13 | + + + | + | + | + + |
| 30 | + + + | + + | + + + | + + + |

+ + + = Wirkung sehr stark, ausgeprägt
+ + = Wirkung stark
+ = Wirkung deutlich.

## Ansprüche

1. Verbindungen der Formel I, deren tautomere Formen der Formel Ia, deren Stereoisomere sowie die Gemische dieser Formen

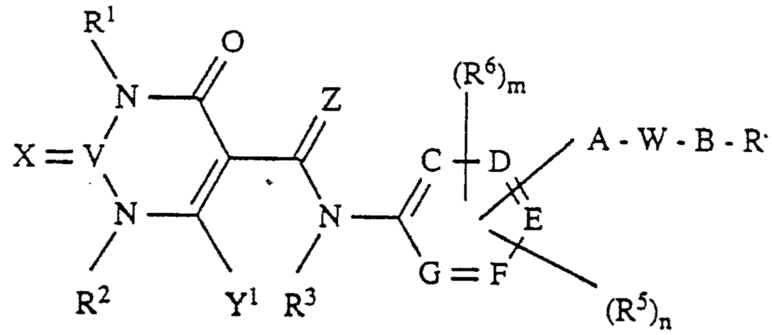

(I),

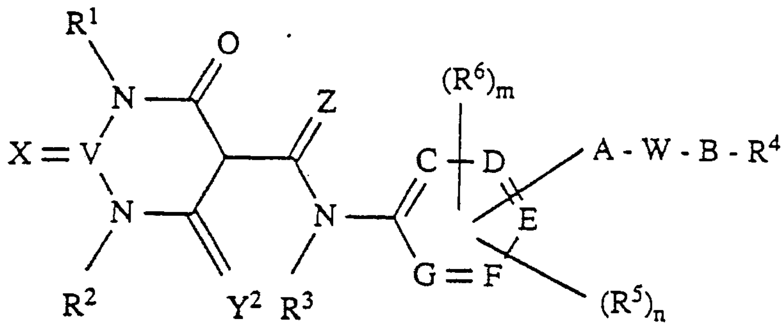

(Ia),

worin

$R^1$,$R^2$ unabhängig voneinander, $(C_1\text{-}C_6)$-Alkyl, $(C_2\text{-}C_6)$-Alkenyl, $(C_2\text{-}C_6)$-Alkinyl, die alle durch ein bis dreizehn Halogenatome und/oder ein bis dreifach durch einen Rest der Gruppe $(C_1\text{-}C_4)$-Alkoxy, $(C_1\text{-}C_4)$-Alkoxycarbonyl, $(C_2\text{-}C_4)$-Alkylsulfenyl, $(C_1\text{-}C_4)$-Alkylsulfinyl, $(C_1\text{-}C_4)$-Alkylsulfonyl, Di$(C_1\text{-}C_4)$-Alkylamino, Cyano, Sulfo, Nitro, Hydroxy, Carboxy, Phenyl oder Phenoxy, wobei die beiden letztgenannten Reste ein- bis dreifach durch einen Rest der Gruppe Halogen, $(C_1\text{-}C_4)$-Alkyl, $(C_1\text{-}C_4)$-Alkoxy, $(C_1\text{-}C_4)$-Alkylcarbonyl, $(C_1\text{-}C_4)$-Alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Halo$(C_1\text{-}C_4)$-alkoxy mit ein bis neun Halogenatomen, Di$(C_1\text{-}C_4)$Alkylamino, Cyano, Sulfo, Nitro substituiert sein können, substituiert sein können;

$(C_1\text{-}C_4)$-Alkoxy, $(C_1\text{-}C_4)$-Alkylsulfonyl, $(C_1\text{-}C_4)$-Alkylcarbonyl, Cyano, Phenylsulfonyl, Phenylcarbonyl, wobei die beiden letztgenannten Reste durch ein bis drei Reste der Gruppe Halogen, $(C_1\text{-}C_4)$-Alkyl, $(C_1\text{-}C_4)$-Alkoxy, Halo$(C_1\text{-}C_4)$-alkoxy mit ein bis neun Halogenatomen oder Nitro substituiert sein können;

Phenyl, das durch ein bis drei Reste aus der Gruppe Halogen, $(C_1\text{-}C_4)$-Alkyl, $(C_1\text{-}C_4)$-Alkoxy, $(C_1\text{-}C_4)$-Alkylcarbonyl, $(C_1\text{-}C_4)$-Alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Halo$(C_1\text{-}C_4)$-alkyl, Halo$(C_1\text{-}C_4)$-alkoxy, mit jeweils ein bis neuen Halogenatomen, Di$(C_1\text{-}C_4)$-Alkylamino, Cyano, Sulfo, Nitro oder Phenoxy, das gegebenenfalls durch ein bis drei Reste der Gruppe Halogen, $(C_1\text{-}C_4)$-Alkyl, $(C_1\text{-}C_4)$-Alkoxy substituiert ist, substituiert sein kann,

$R^3$ Wasserstoff, $(C_1\text{-}C_6)$-Alkyl; Phenyl, das durch ein bis drei Reste aus der Gruppe Halogen, $(C_1\text{-}C_4)$-Alkyl, $(C_1\text{-}C_4)$-Alkoxy, $(C_1\text{-}C_4)$-Alkylcarbonyl, $(C_1\text{-}C_4)$-Alkoxycarbonyl, $(C_1\text{-}C_4)$-Alkylsulfenyl, $(C_1\text{-}C_4)$-Alkylsulfinyl, $(C_1\text{-}C_4)$-Alkylsulfonyl, Halo$(C_1\text{-}C_4)$-alkyl, Halo$(C_1\text{-}C_4)$-alkoxy, mit jeweils ein bis neun Halogenatomen, Di$(C_1\text{-}C_4)$-Alkylamino, Cyano, Sulfo, Nitro oder Phenoxy, das gegebenenfalls durch ein bis drei Reste der Gruppe Halogen, $(C_1\text{-}C_4)$-Alkyl, $(C_1\text{-}C_4)$-Alkoxy substituiert ist, substituiert sein kann,

$R^4$ Wasserstoff, $(C_1\text{-}C_6)$-Alkyl, $(C_2\text{-}C_6)$-Alkenyl, $(C_2\text{-}C_6)$-Alkinyl, die durch ein- bis neun Halogenatome und/oder ein- bis dreifach durch einen Rest der Gruppe $(C_1\text{-}C_6)$-Alkoxy, Halo$(C_1\text{-}C_6)$-alkoxy, $(C_1\text{-}C_6)$-Alkoxycarbonyl, Halo$(C_1\text{-}C_6)$-alkoxycarbonyl, $(C_1\text{-}C_6)$-Alkylsulfenyl, $(C_1\text{-}C_6)$-Alkylsulfinyl, $(C_1\text{-}C_6)$-Alkylsulfonyl, Di$(C_1\text{-}C_6)$-Alkylamino, Cyano, Sulfo, Nitro, Hydroxy, Carboxy, Phenyl oder Phenoxy, wobei die beiden letztgenannten Reste ein- bis dreifach durch einen Rest der Gruppe Halogen, $(C_1\text{-}C_4)$-Alkyl, Halo$(C_1\text{-}C_4)$-

alkyl mit ein bis neun Halogenatomen, ($C_1$-$C_4$)-Alkoxy, ($C_1$-$C_4$)-Alkylcarbonyl, Halo($C_1$-$C_4$)-alkylcarbonyl, ($C_1$-$C_4$)-Alkoxycarbonyl, Halo($C_1$-$C_4$)-alkoxycarbonyl, ($C_1$-$C_4$)-Alkylsulfenyl, ($C_1$-$C_4$)-Alkylsulfinyl, ($C_1$-$C_4$)-Alkylsulfonyl, Sulfato, Halo($C_1$-$C_4$)-alkoxy mit ein bis neun Halogenatomen, Di($C_1$-$C_4$)-Alkylamino, Cyano, Sulfo, Nitro substituiert sein können, substituiert sein können; ($C_1$-$C_6$)-Alkylsulfonyl, ($C_1$-$C_6$)-Alkylcarbonyl, Halo($C_1$-$C_6$)-alkylsulfonyl, Halo($C_1$-$C_6$)-alkylcarbonyl, Mono-, Di($C_1$-$C_4$)-Alkylcarbamoyl, Cyano, Phenylsulfonyl, Phenylcarbonyl, Phenylcarbamoyl, wobei die drei letztgenannten Reste durch ein bis drei Reste der Gruppe Halogen, ($C_1$-$C_4$)-Alkyl, Halo($C_1$-$C_4$)-alkyl, ($C_1$-$C_4$)-Alkoxy, Halo($C_1$-$C_4$)-alkoxy, ($C_1$-$C_4$)-Alkylcarbonyl, Halo($C_1$-$C_4$)-alkylcarbonyl, ($C_1$-$C_4$)-Alkoxycarbonyl, Halo($C_1$-$C_4$)-alkoxycarbonyl, ($C_1$-$C_4$)-Alkylsulfenyl, ($C_1$-$C_4$)-Alkylsulfinyl, ($C_1$-$C_4$)-Alkylsulfonyl, Di($C_1$-$C_4$)-Alkylamino, Cyano, Sulfo, Nitro substituiert sein können;

Phenyl, das durch ein bis drei Reste aus der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo($C_1$-$C_4$)-alkoxy, mit jeweils ein bis neun Halogenatomen, Di($C_1$-$C_4$)-Alkylamino, Cyano, Sulfo, Nitro oder Phenoxy, das gegebenenfalls durch ein bis drei Reste der Gruppe Halogen, ($C_1$-$C_4$)-Alkyl, ($C_1$-$C_4$)-Alkoxy substituiert ist, substituiert sein kann,

$R^5$,$R^6$ unabhängig voneinander Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo-($C_1$-$C_4$)-alkoxy mit jeweils ein bis neun Halogenatomen, Di($C_1$-$C_4$)-Alkylamino, Cyano, Sulfo, Nitro,

A,X,Z unabhängig voneinander Sauerstoff, Schwefel, Sulfinyl, Sulfonyl oder Stickstoff, der durch ($C_1$-$C_4$)-Alkyl substituiert sein kann,

B Sauerstoff, Schwefel, Sulfinyl, Sulfonyl oder Stickstoff, der durch ($C_1$-$C_4$)-Alkyl substituiert sein kann; oder eine Doppelbindung,

C,D,E,F,G unabhängig voneinander Kohlenstoff oder Stickstoff,

V Kohlenstoff oder Phosphor, der durch ($C_1$-$C_6$)-Alkoxy, ($C_1$-$C_4$)-Alkylthio, Phenoxy oder Phenylthio, die beide ein bis drei Reste aus der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo($C_1$-$C_4$)-alkoxy mit jeweils ein bis neun Halogenatomen, Di($C_1$-$C_4$)-Alkylamino, Cyano, Sulfo, Nitro tragen können, substituiert ist,

W ($C_1$-$C_{10}$)-Alkylen, Halo($C_1$-$C_{10}$)-Alkylen, die durch ein bis fünf Reste der Gruppe ($C_1$-$C_4$)-Alkyl, Halo-($C_1$-$C_4$)-Alkyl, ($C_1$-$C_4$)-Alkoxy, Halo($C_1$-$C_4$)-Alkoxy, Cyano oder Nitro substituiert sein können,

$Y^1$ H, Halogen, ($C_1$-$C_4$)-Alkyl, Amino, ($C_1$-$C_4$)-Alkylamino, Di($C_1$-$C_4$)-Alkylamino, Hydroxy,

$Y^2$ Sauerstoff, Schwefel oder Stickstoff, der durch ($C_1$-$C_4$)-Alkyl substituiert sein kann, und

n,m unabhängig voneinander die Zahlen 0 bis 4 bedeuten, sowie deren in der Landwirtschaft einsetzbaren oder physiologisch verträglichen Salze.

2. Verbindungen der Formeln I und Ia gemäß Anspruch 1, worin

$R^1$,$R^2$ unabhängig voneinander, ($C_1$-$C_6$)-Alkyl, ($C_2$-$C_6$)-Alkenyl, ($C_2$-$C_6$)-Alkinyl, die durch ein bis dreizehn Halogenatome und/oder ein bis dreifach durch einen Rest der Gruppe ($C_1$-$C_4$)-Alkoxy, ($C_1$-$C_4$)-Alkoxycarbonyl, ($C_1$-$C_4$)-Alkylsulfenyl, Di($C_1$-$C_4$)-Alkylamino, Cyano, Sulfo, Nitro, Hydroxy, Carboxy, Phenyl oder Phenoxy, wobei di beiden letztgenannten Reste ein- bis dreifach durch einen Rest der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkoxy mit ein bis neun Halogenatomen, Di($C_1$-$C_4$)-Alkylamino, Cyano, Sulfo, Nitro substituiert sein können, substituiert sein können,

$R^3$ Wasserstoff, ($C_1$-$C_6$)-Alkyl,

$R^4$ Wasserstoff, ($C_1$-$C_6$)-Alkyl,($C_2$-$C_6$)-Alkenyl, ($C_2$-$C_6$)-Alkinyl, die durch ein- bis neun Halogenatome und/oder ein- bis dreifach durch einen Rest der Gruppe ($C_1$-$C_6$)-Alkoxy, Halo($C_1$-$C_6$)-alkoxy, ($C_1$-$C_6$)-Alkoxycarbonyl, Halo($C_1$-$C_6$)-alkoxycarbonyl, ($C_1$-$C_6$)-Alkylsulfenyl, Cyano, Sulfo, Nitro, Hydroxy, Carboxy, Phenyl oder Phenoxy, wobei die beiden letztgenannten Reste ein- bis dreifach durch einen Rest der Gruppe Halogen, ($C_1$-$C_4$)-Alkyl, Halo($C_1$-$C_4$)-alkyl, ($C_1$-$C_4$)-Alkoxy, Halo($C_1$-$C_4$)-alkoxy, ($C_1$-$C_4$)-Alkylcarbonyl, Halo($C_1$-$C_4$)-alkylcarbonyl, ($C_1$-$C_4$)-Alkoxycarbonyl, Halo($C_1$-$C_4$)-alkoxycarbonyl, ($C_1$-$C_4$)-Alkylsulfenyl, ($C_1$-$C_4$)-Alkylsulfinyl, ($C_1$-$C_4$)-Alkylsulfonyl, Di($C_1$-$C_4$)-Alkylamino, Cyano, Sulfo, Nitro substituiert sein können, substituiert sein können;

($C_1$-$C_6$)-Alkylsulfonyl, ($C_1$-$C_6$)-Alkylcarbonyl, Halo($C_1$-$C_6$)-alkylsulfonyl, Halo($C_1$-$C_6$)-alkylcarbonyl, Mono-, Di-($C_1$-$C_4$)-Alkylcarbamoyl, Cyano, Phenylsulfonyl, Phenylcarbonyl, Phenylcarbamoyl, wobei die drei letztgenannten Reste durch ein bis drei Reste der Gruppe Halogen, ($C_1$-$C_4$)-Alkyl, Halo($C_1$-$C_4$)-alkyl, ($C_1$-$C_4$)-Alkoxy, Halo($C_1$-$C_4$)-alkoxy, ($C_1$-$C_4$)-Alkylcarbonyl, Halo($C_1$-$C_4$)-alkylcarbonyl ($C_1$-$C_4$)-Alkoxycarbonyl, Halo-($C_1$-$C_4$)-alkoxycarbonyl, ($C_1$-$C_4$)-Alkylsulfenyl, ($C_1$-$C_4$)-Alkylsulfinyl, ($C_1$-$C_4$)-Alkylsulfonyl, Di($C_1$-$C_4$)-Alkylamino, Cyano, Sulfo, Nitro substituiert sein können,

$R^5$,$R^6$ unabhängig voneinander Halogen, ($C_1$-$C_4$)-Alkyl, ($C_1$-$C_4$)-Alkoxy, ($C_1$-$C_4$)-Alkylcarbonyl, ($C_1$-$C_4$)-

Alkoxycarbonyl, ($C_1$-$C_4$)-Alkylsulfenyl, Halo($C_1$-$C_4$)-alkoxy mit ein bis neun Halogenatomen, Di($C_1$-$C_4$)-Alkylamino, Cyano, Sulfo, Nitro,

A,X,Z unabhängig voneinander Sauerstoff, Schwefel, Sulfinyl oder Sulfonyl,

B Sauerstoff, Schwefel, Sulfinyl, Sulfonyl oder Stickstoff, der durch ($C_1$-$C_4$)-Alkyl substituiert sein kann; oder eine Doppelbindung,

C,D,E,F,G unabhängig voneinander Kohlenstoff oder Stickstoff,

V Kohlenstoff oder Phosphor, der durch ($C_1$-$C_6$)-Alkoxy, ($C_1$-$C_4$)-Alkylthio, Phenoxy oder Phenylthio, die beide ein bis drei Reste aus der Gruppe Halogen ($C_1$-$C_4$)-Alkyl, ($C_1$-$C_4$)-Alkoxy, Halo($C_1$-$C_4$)-alkoxy, Cyano, Sulfo, Nitro tragen können, substituiert ist,

W ($C_1$-$C_{10}$)-Alkylen, Halo($C_1$-$C_{10}$)-Alkylen, die durch ein bis fünf Reste der Gruppe ($C_1$-$C_4$)-Alkyl, Halo-($C_1$-$C_4$)-alkyl, ($C_1$-$C_4$)-Alkoxy, Halo($C_1$-$C_4$)-alkoxy, Cyano oder Nitro substituiert sein können,

$Y^1$ H, Halogen, ($C_1$-$C_4$)-Alkyl, Amino, ($C_1$-$C_4$)-Alkylamino, Di($C_1$-$C_4$)-Alkylamino, Hydroxy,

$Y^2$ Sauerstoff, Schwefel oder Stickstoff, der durch ($C_1$-$C_4$)-Alkyl substituiert sein kann und

n,m unabhängig voneinander die Zahlen 0 bis 4 bedeuten, sowie deren in der Landwirtschaft einsetzbaren oder physiolgisch verträglichen Salze.

3. Verbindungen der Formeln I und Ia gemäß Anspruch 1 oder 2, worin

$R^1$, $R^2$ unabhängig voneinander, ($C_1$-$C_6$)-Alkyl, ($C_2$-$C_6$)-Alkenyl, ($C_2$-$C_6$)-Alkinyl, die durch ein bis dreizehn Halogenatome und/oder ($C_1$-$C_4$)-Alkoxy ($C_1$-$C_4$)-Alkoxycarbonyl, ($C_1$-$C_4$)-Alkylsulfenyl substituiert sein können,

$R^3$ Wasserstoff, ($C_1$-$C_6$)-Alkyl,

$R^4$ Wasserstoff, ($C_1$-$C_6$)-Alkyl, ($C_2$-$C_6$)-Alkenyl, ($C_2$-$C_6$)-Alkinyl, die durch ein- bis neun Halogenatome und/oder ein- bis dreifach durch einen Rest der Gruppe ($C_1$-$C_6$)-Alkoxy, Halo($C_1$-$C_6$)-alkoxy, ($C_1$-$C_6$)-Alkoxycarbonyl, Halo($C_1$-$C_6$)-alkoxycarbonyl, ($C_1$-$C_6$)-Alkylsulfenyl, ($C_1$-$C_6$)-Alkylsulfinyl, Di($C_1$-$C_6$)-Alkylamino, Cyano, Sulfo, Nitro, Hydroxy, Carboxy substituiert sein können; ($C_1$-$C_6$)-Alkylsulfonyl, ($C_1$-$C_6$)-Alkylcarbonyl, Halo($C_1$-$C_6$)-alkylsulfonyl, Halo($C_1$-$C_6$)-alkylcarbonyl, Mono-, Di-($C_1$-$C_4$)-Alkylcarbamoyl,

$R^5$,$R^6$ unabhängig voneinander Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, Halo($C_1$-$C_4$)-alkoxy mit ein bis neun Halogenatomen, Di($C_1$-$C_4$)-Alkylamino, Cyano, Sulfo, Nitro,

A,X,Z unabhängig voneinander Sauerstoff, Schwefel,

B Sauerstoff, Schwefel oder Stickstoff, der durch ($C_1$-$C_4$)-Alkyl substituiert sein kann; oder eine Doppelbindung

C,D,E,F,G unabhängig voneinander Kohlenstoff oder Stickstoff

V Kohlenstoff

W ($C_1$-$C_{10}$)-Alkylen, Halo($C_1$-$C_{10}$)-Alkylen, die durch ein bis fünf Reste der Gruppe ($C_1$-$C_4$)-Alkyl, Halo-($C_1$-$C_4$)-alkyl, ($C_1$-$C_4$)-Alkoxy oder Halo($C_1$-$C_4$)-alkoxy substituiert sein können,

$Y^1$ H, Amino oder Hydroxy

$Y^2$ Sauerstoff, Schwefel oder Stickstoff, der durch ($C_1$-$C_4$)-Alkyl substituiert sein kann und

n,m unabhängig voneinander die Zahlen 0 bis 4 bedeuten, sowie deren in der Landwirtschaft einsetzbaren oder physiologisch verträglichen Salze.

4. Verfahren zur Herstellung von Verbindungen der Formeln I und Ia gemäß einem oder mehreren der Ansprüche 1 bis 3, dadurch gekennzeichnet, daß man

a) eine Verbindung der Formel (II) oder (III),

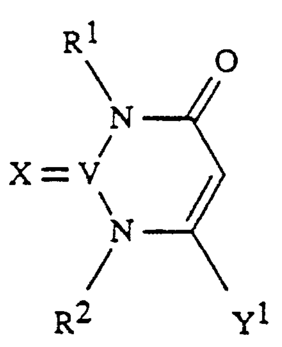
(II),

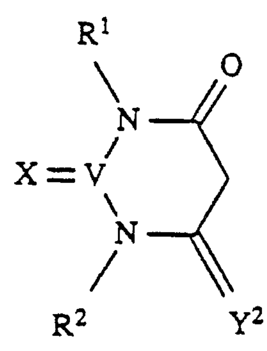
(III),

worin $R^1$,$R^2$,V,X,$Y^1$ und $Y^2$ dieselbe Bedeutung wie in Formeln (I) und (Ia) besitzen, mit einer Verbindung der

Formel (IV),

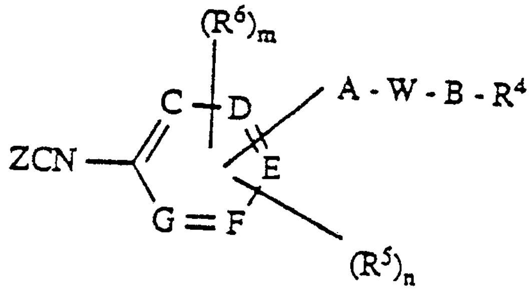

IV

worin A,B,C,D,E,F,G,W,Z,$R^4$,$R^5$,$R^6$,n und m dieselbe Bedeutung wie in Formeln (I) und (Ia) besitzen umsetzt oder

b) eine Verbindung der Formel (V) oder (VI),

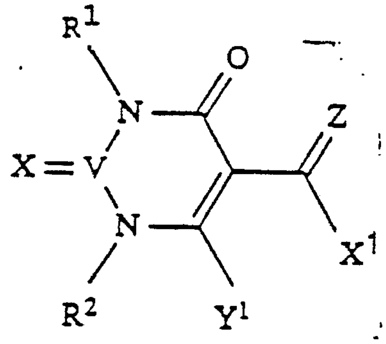

(V),

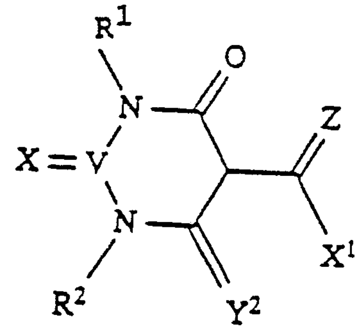

(VI),

worin $R^1$,$R^2$,V,X,$Y^1$,$Y^2$ und Z dieselbe Bedeutung wie in Formeln (I) und (Ia) besitzen und $X^1$ für eine nukleofuge Abgangsgruppe wie z.B. Halogen ($C_1$-$C_6$)-Alkoxy, ($C_1$-$C_6$)-Alkylthio, Phenyl, Imidazolyl oder Triazolyl steht, mit einer Verbindung der Formel (VII),

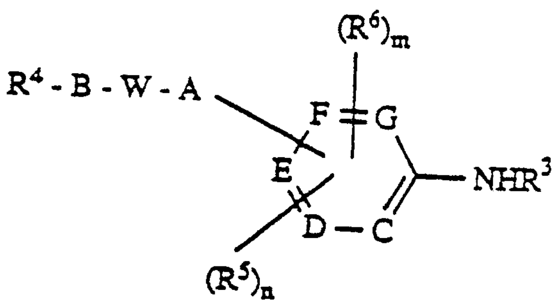

(VII),

worin A,B,C,D,E,F,G,W,$R^3$,$R^4$,$R^5$,$R^6$,n und m die selbe Bedeutung wie in Formeln (I) und (Ia) besitzen, umsetzt.

5. Verbindungen der Formel IV gemäß Anspruch 4.

6. Verbindungen der Formel VII gemäß Anspruch 4.

7. Insektizide, akarizide oder nematozide Mittel, dadurch gekennzeichnet, daß sie eine wirksame Menge einer Verbindung der Formel I oder der Formel Ia enthalten.

8. Verwendung von Verbindungen der Formel I oder der Formel Ia zur Bekämpfung von Schadinsekten, Akariden oder Nematoden.

9. Verfahren zur Bekämpfung von Schadinsekten, Akariden oder Nematoden, dadurch gekennzeichnet, daß man auf diese oder die von ihnen befallenen Pflanzen, Flächen oder Substrate eine wirksame Menge einer Verbindung der Formel I oder Ia appliziert.

10. Tierarzneimittel, dadurch gekennzeichnet, daß es eine Verbindung der Formel I oder der Formel Ia nach einem oder mehreren der Ansprüche 1-3 enthält oder aus dieser besteht.

11. Mittel zur Bekämpfung von Ekto- und Endoparasiten bei Tieren, dadurch gekennzeichnet, daß es eine Verbindung der Formel I oder der Formel Ia nach einem oder mehreren der Ansprüche 1-3 neben geeigneten Formulierungshilfsmitteln enthält.

12. Verfahren zur Bekämpfung von Ekto- und Endoparasiten bei Tieren, dadurch gekennzeichnet, daß man diese mit einem Mittel gemäß Anspruch 11 behandelt.

Patentansprüche für folgenden Vertragsstaat: ES

1. Verfahren zur Herstellung von Verbindungen der Formel I, deren tautomere Formen der Formel Ia, deren Stereoisomere sowie die Gemische dieser Formen

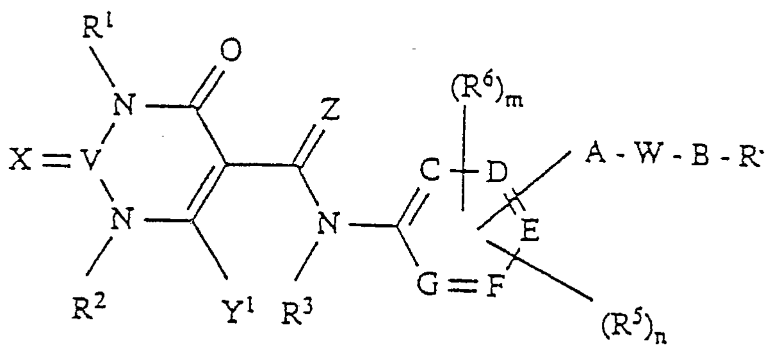

(I) ,

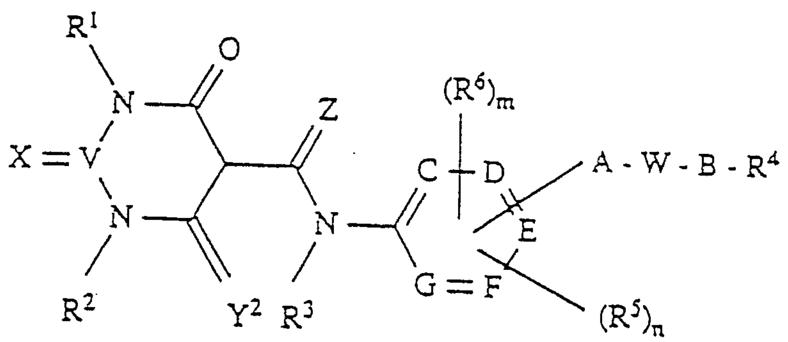

(Ia) ,

worin

$R^1$,$R^2$ unabhängig voneinander, ($C_1$-$C_6$)-Alkyl, ($C_2$-$C_6$)-Alkenyl, ($C_2$-$C_6$)-Alkinyl, die alle durch ein bis dreizehn Halogenatome und/oder ein bis dreifach durch einen Rest der Gruppe ($C_1$-$C_4$)-Alkoxy, ($C_1$-$C_4$)-Alkoxycarbonyl, ($C_2$-$C_4$)-Alkylsulfenyl, ($C_1$-$C_4$)-Alkylsulfinyl, ($C_1$-$C_4$)-Alkylsulfonyl, Di($C_1$-$C_4$)-Alkylamino, Cyano, Sulfo, Nitro, Hydroxy, Carboxy, Phenyl oder Phenoxy, wobei die beiden letztgenannten Reste ein- bis dreifach durch einen Rest der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkoxy mit ein bis neun Halogenatomen, Di($C_1$-$C_4$)Alkylamino, Cyano, Sulfo, Nitro substituiert sein können, substituiert sein können;

($C_1$-$C_4$)-Alkoxy, ($C_1$-$C_4$)-Alkylsulfonyl, ($C_1$-$C_4$)-Alkylcarbonyl, Cyano, Phenylsulfonyl, Phenylcarbonyl, wobei die beiden letztgenannten Reste durch ein bis drei Reste der Gruppe Halogen, ($C_1$-$C_4$)-Alkyl, ($C_1$-$C_4$)-Alkoxy, Halo($C_1$-$C_4$)-alkoxy mit ein bis neun Halogenatomen oder Nitro substituiert sein können;

Phenyl, das durch ein bis drei Reste aus der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo($C_1$-$C_4$)-alkoxy, mit jeweils ein bis neuen Halogenatomen, Di($C_1$-$C_4$)-Alkylamino, Cyano, Sulfo, Nitro oder Phenoxy, das gegebenenfalls durch ein bis drei Reste der Gruppe Halogen, ($C_1$-$C_4$)-Alkyl, ($C_1$-$C_4$)-Alkoxy substituiert ist, substituiert sein kann,

$R^3$ Wasserstoff, ($C_1$-$C_6$)-Alkyl; Phenyl, das durch ein bis drei Reste aus der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo($C_1$-$C_4$)-alkoxy, mit jeweils ein bis neun Halogenatomen,

Di($C_1$-$C_4$)-Alkylamino, Cyano, Sulfo, Nitro oder Phenoxy, das gegebenenfalls durch ein bis drei Reste der Gruppe Halogen, ($C_1$-$C_4$)-Alkyl, ($C_1$-$C_4$)-Alkoxy substituiert ist, substituiert sein kann,

$R^4$ Wasserstoff, ($C_1$-$C_6$)-Alkyl, ($C_2$-$C_6$)-Alkenyl, ($C_2$-$C_6$)-Alkinyl, die durch ein- bis neun Halogenatome und/oder ein- bis dreifach durch einen Rest der Gruppe ($C_1$-$C_6$)-Alkoxy, Halo($C_1$-$C_6$)-alkoxy, ($C_1$-$C_6$)-Alkoxycarbonyl, Halo($C_1$-$C_6$)-alkoxycarbonyl, ($C_1$-$C_6$)-Alkylsulfenyl, ($C_1$-$C_6$)-Alkylsulfinyl, ($C_1$-$C_6$)-Alkylsulfonyl, Di($C_1$-$C_6$)-Alkylamino, Cyano, Sulfo, Nitro, Hydroxy, Carboxy, Phenyl oder Phenoxy, wobei die beiden letztgenannten Reste ein- bis dreifach durch einen Rest der Gruppe Halogen, ($C_1$-$C_4$)-Alkyl, Halo($C_1$-$C_4$)-alkyl mit ein bis neun Halogenatomen, ($C_1$-$C_4$)-Alkoxy, ($C_1$-$C_4$)-Alkylcarbonyl, Halo($C_1$-$C_4$)-alkylcarbonyl, ($C_1$-$C_4$)-Alkoxycarbonyl, Halo($C_1$-$C_4$)-alkoxycarbonyl, ($C_1$-$C_4$)-Alkylsulfenyl, ($C_1$-$C_4$)-Alkylsulfinyl, ($C_1$-$C_4$)-Alkylsulfonyl, Sulfato, Halo($C_1$-$C_4$)-alkoxy mit ein bis neun Halogenatomen, Di($C_1$-$C_4$)-Alkylamino, Cyano, Sulfo, Nitro substituiert sein können, substituiert sein können; ($C_1$-$C_6$)-Alkylsulfonyl, ($C_1$-$C_6$)-Alkylcarbonyl, Halo($C_1$-$C_6$)-alkylsulfonyl, Halo($C_1$-$C_6$)-alkylcarbonyl, Mono-, Di($C_1$-$C_4$)-Alkylcarbamoyl, Cyano, Phenylsulfonyl, Phenylcarbonyl, Phenylcarbamoyl, wobei die drei letztgenannten Reste durch ein bis drei Reste der Gruppe Halogen, ($C_1$-$C_4$)-Alkyl, Halo($C_1$-$C_4$)-alkyl, ($C_1$-$C_4$)-Alkoxy, Halo($C_1$-$C_4$)-alkoxy, ($C_1$-$C_4$)-Alkylcarbonyl, Halo($C_1$-$C_4$)-alkylcarbonyl, ($C_1$-$C_4$)-Alkoxycarbonyl, Halo($C_1$-$C_4$)-alkoxycarbonyl, ($C_1$-$C_4$)-Alkylsulfenyl, ($C_1$-$C_4$)-Alkylsulfinyl, ($C_1$-$C_4$)-Alkylsulfonyl, Di($C_1$-$C_4$)-Alkylamino, Cyano, Sulfo, Nitro substituiert sein können;

Phenyl, das durch ein bis drei Reste aus der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo($C_1$-$C_4$)-alkoxy, mit jeweils ein bis neun Halogenatomen, Di($C_1$-$C_4$)-Alkylamino, Cyano, Sulfo, Nitro oder Phenoxy, das gegebenenfalls durch ein bis drei Reste der Gruppe Halogen, ($C_1$-$C_4$)-Alkyl, ($C_1$-$C_4$)-Alkoxy substituiert ist, substituiert sein kann,

$R^5$,$R^6$ unabhängig voneinander Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo-($C_1$-$C_4$) alkoxy mit jeweils ein bis neun Halogenatomen, Di($C_1$-$C_4$)-Alkylamino, Cyano, Sulfo, Nitro,

A,X,Z unabhängig voneinander Sauerstoff, Schwefel, Sulfinyl, Sulfonyl oder Stickstoff, der durch ($C_1$-$C_4$)-Alkyl substituiert sein kann,

B Sauerstoff, Schwefel, Sulfinyl, Sulfonyl oder Stickstoff, der durch ($C_1$-$C_4$)-Alkyl substituiert sein kann; oder eine Doppelbindung,

C,D,E,F,G unabhängig voneinander Kohlenstoff oder Stickstoff,

V Kohlenstoff oder Phosphor, der durch ($C_1$-$C_6$)-Alkoxy, ($C_1$-$C_4$)-Alkylthio, Phenoxy oder Phenylthio, die beide ein bis drei Reste aus der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, ($C_1$-$C_4$)-Alkylsulfinyl, ($C_1$-$C_4$)-Alkylsulfonyl, Halo($C_1$-$C_4$)-alkyl, Halo($C_1$-$C_4$)-alkoxy mit jeweils ein bis neun Halogenatomen, Di($C_1$-$C_4$)-Alkylamino, Cyano, Sulfo, Nitro tragen können, substituiert ist,

W ($C_1$-$C_{10}$)-Alkylen, Halo($C_1$-$C_{10}$)-Alkylen, die durch ein bis fünf Reste der Gruppe ($C_1$-$C_4$)-Alkyl, Halo-($C_1$-$C_4$)-Alkyl, ($C_1$-$C_4$)-Alkoxy, Halo($C_1$-$C_4$)-Alkoxy, Cyano oder Nitro substituiert sein können,

$Y^1$ H, Halogen, ($C_1$-$C_4$)-Alkyl, Amino, ($C_1$-$C_4$)-Alkylamino, Di($C_1$-$C_4$)-Alkylamino, Hydroxy,

$Y^2$ Sauerstoff, Schwefel oder Stickstoff, der durch ($C_1$-$C_4$)-Alkyl substituiert sein kann, und

n,m unabhängig voneinander die Zahlen 0 bis 4 bedeuten, sowie deren in der Landwirtschaft einsetzbaren oder physiologisch verträglichen Salze, dadurch gekennzeichnet, daß man

a) eine Verbindung der Formel (II) oder (III),

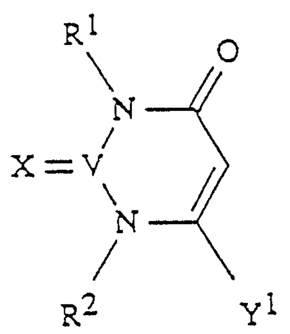 (II) ,

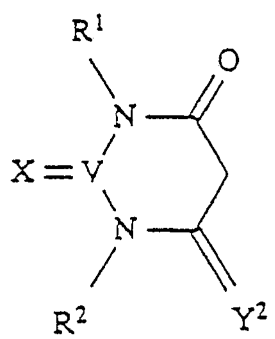 (III) ,

worin $R^1$,$R^2$,V,X,$Y^1$ und $Y^2$ dieselbe Bedeutung wie in Formeln (I) und (Ia) besitzen, mit einer Verbindung der

Formel (IV),

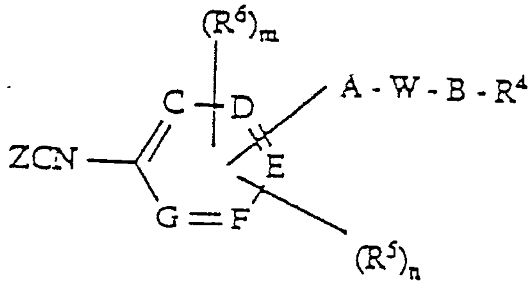

IV

worin A,B,C,D,E,F,G,W,Z,$R^4$,$R^5$,$R^6$,n und m dieselbe Bedeutung wie in Formeln (I) und (Ia) besitzen umsetzt oder

b) eine Verbindung der Formel (V) oder (VI),

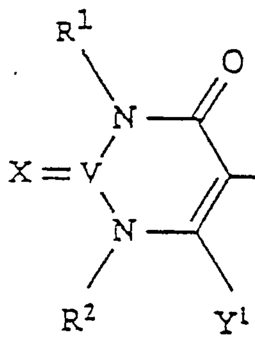

(V),

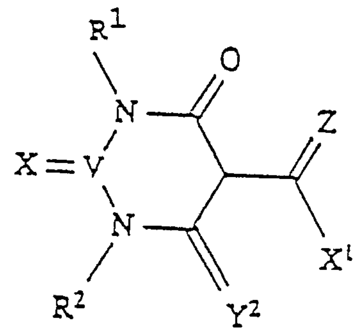

(VI),

worin $R^1$,$R^2$,V,X,$Y^1$,$Y^2$ und Z dieselbe Bedeutung wie in Formeln (I) und (Ia) besitzen und $X^1$ für eine nukleofuge Abgangsgruppe wie z.B. Halogen ($C_1$-$C_6$)-Alkoxy, ($C_1$-$C_6$)-Alkylthio, Phenyl, Imidazolyl oder Triazolyl steht, mit einer Verbindung der Formel (VII),

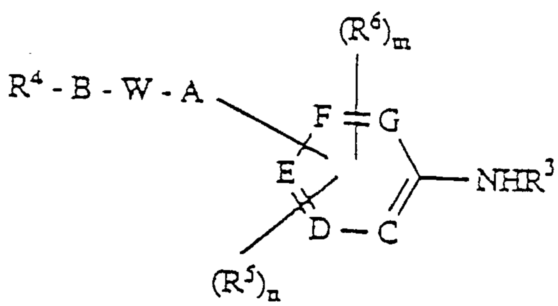

(VII),

worin A,B,C,D,E,F,G,W,$R^3$,$R^4$,$R^5$,$R^6$,n und m dieselbe Bedeutung wie in Formeln (I) und (Ia) besitzen, umsetzt.

2. Verfahren gemäß Anspruch 1, dadurch gekennzeichnet, daß in Formeln I und Ia

$R^1$,$R^2$ unabhängig voneinander, ($C_1$-$C_6$)-Alkyl, ($C_2$-$C_6$)-Alkenyl, ($C_2$-$C_6$)-Alkinyl, die durch ein bis dreizehn Halogenatome und/oder ein bis dreifach durch einen Rest der Gruppe ($C_1$-$C_4$)-Alkoxy, ($C_1$-$C_4$)-Alkoxycarbonyl, ($C_1$-$C_4$)-Alkylsulfenyl, Di($C_1$-$C_4$)-Alkylamino, Cyano, Sulfo, Nitro, Hydroxy, Carboxy, Phenyl oder Phenoxy, wobei die beiden letztgenannten Reste ein- bis dreifach durch einen Rest der Gruppe Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl ($C_1$-$C_4$)-Alkylsulfinyl, ($C_1$-$C_4$)-Alkylsulfonyl, Halo($C_1$-$C_4$)-alkoxy mit ein bis neun Halogenatomen, Di($C_1$-$C_4$)-Alkylamino, Cyano, Sulfo, Nitro substituiert sein können, substituiert sein können,

$R^3$ Wasserstoff, ($C_1$-$C_6$)-Alkyl,

$R^4$ Wasserstoff, ($C_1$-$C_6$)-Alkyl,($C_2$-$C_6$)-Alkenyl, ($C_2$-$C_6$)-Alkinyl, die durch ein- bis neun Halogenatome und/oder ein- bis dreifach durch einen Rest der Gruppe ($C_1$-$C_6$)-Alkoxy, Halo($C_1$-$C_6$)-alkoxy, ($C_1$-$C_6$)-

Alkoxycarbonyl, Halo($C_1$-$C_6$)-alkoxycarbonyl, ($C_1$-$C_6$)-Alkylsulfenyl, Cyano, Sulfo, Nitro, Hydroxy, Carboxy, Phenyl oder Phenoxy, wobei die beiden letztgenannten Reste ein- bis dreifach durch einen Rest der Gruppe Halogen, ($C_1$-$C_4$)-Alkyl, Halo($C_1$-$C_4$)-alkyl, ($C_1$-$C_4$)-Alkoxy, Halo($C_1$-$C_4$)-alkoxy, ($C_1$-$C_4$)-Alkylcarbonyl, Halo($C_1$-$C_4$)-alkylcarbonyl, ($C_1$-$C_4$)-Alkoxycarbonyl, Halo($C_1$-$C_4$)-alkoxycarbonyl, ($C_1$-$C_4$)-Alkylsulfenyl ($C_1$-$C_4$)-Alkylsulfinyl, ($C_1$-$C_4$)-Alkylsulfonyl, Di($C_1$-$C_4$)-Alkylamino, Cyano, Sulfo, Nitro substituiert sein können, substituiert sein können;

($C_1$-$C_6$)-Alkylsulfonyl, ($C_1$-$C_6$)-Alkylcarbonyl, Halo($C_1$-$C_6$)-alkylsulfonyl, Halo($C_1$-$C_6$)-alkylcarbonyl, Mono-, Di-($C_1$-$C_4$)-Alkylcarbamoyl, Cyano, Phenylsulfonyl, Phenylcarbonyl, Phenylcarbamoyl, wobei die drei letztgenannten Reste durch ein bis drei Reste der Gruppe Halogen, ($C_1$-$C_4$)-Alkyl, Halo($C_1$-$C_4$)-alkyl, ($C_1$-$C_4$)-Alkoxy, Halo($C_1$-$C_4$)-alkoxy, ($C_1$-$C_4$)-Alkylcarbonyl, Halo($C_1$-$C_4$)-alkylcarbonyl ($C_1$-$C_4$)-Alkoxycarbonyl, Halo-($C_1$-$C_4$)-alkoxycarbonyl, ($C_1$-$C_4$)-Alkylsulfenyl, ($C_1$-$C_4$)-Alkylsulfinyl, ($C_1$-$C_4$)-Alkylsulfonyl, Di($C_1$-$C_4$)-Alkylamino, Cyano, Sulfo, Nitro substituiert sein können,

$R^5$,$R^6$ unabhängig voneinander Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, Halo($C_1$-$C_4$)-alkoxy mit ein bis neun Halogenatomen, Di($C_1$-$C_4$)-Alkylamino, Cyano, Sulfo, Nitro,

A,X,Z unabhängig voneinander Sauerstoff, Schwefel, Sulfinyl oder Sulfonyl,

B Sauerstoff, Schwefel, Sulfinyl, Sulfonyl oder Stickstoff, der durch ($C_1$-$C_4$)-Alkyl substituiert sein kann; oder eine Doppelbindung,

C,D,E,F,G unabhängig voneinander Kohlenstoff oder Stickstoff,

V Kohlenstoff oder Phosphor, der durch ($C_1$-$C_6$)-Alkoxy, ($C_1$-$C_4$)-Alkylthio, Phenoxy oder Phenylthio, die beide ein bis drei Reste aus der Gruppe Halogen ($C_1$-$C_4$)-Alkyl, ($C_1$-$C_4$)-Alkoxy, Halo($C_1$-$C_4$)-alkoxy, Cyano, Sulfo, Nitro tragen können, substituiert ist,

W ($C_1$-$C_{10}$)-Alkylen, Halo($C_1$-$C_{10}$)-Alkylen, die durch ein bis fünf Reste der Gruppe ($C_1$-$C_4$)-Alkyl, Halo-($C_1$-$C_4$)-alkyl, ($C_1$-$C_4$)-Alkoxy, Halo($C_1$-$C_4$)-alkoxy, Cyano oder Nitro substituiert sein können,

$Y^1$ H, Halogen, $C_1$-$C_4$)-Alkyl, Amino, ($C_1$-$C_4$)-Alkylamino, Di($C_1$-$C_4$)-Alkylamino, Hydroxy,

$Y^2$ Sauerstoff, Schwefel oder Stickstoff, der durch ($C_1$-$C_4$)-Alkyl substituiert sein kann und

n,m unabhängig voneinander die Zahlen 0 bis 4 bedeuten, sowie deren in der Landwirtschaft einsetzbaren oder physiolgisch verträglichen Salze.

3. Verfahren gemäß Anspruch 1 oder 2, dadurch gekennzeichnet, daß man in Formeln I und Ia

$R^1$, $R^2$ unabhängig voneinander, ($C_1$-$C_6$)-Alkyl, ($C_2$-$C_6$)-Alkenyl, ($C_2$-$C_6$)-Alkinyl, die durch ein bis dreizehn Halogenatome und/oder ($C_1$-$C_4$)-Alkoxy ($C_1$-$C_4$)-Alkoxycarbonyl, ($C_1$-$C_4$)-Alkylsulfenyl substituiert sein können,

$R^3$ Wasserstoff, ($C_1$-$C_6$)-Alkyl,

$R^4$ Wasserstoff, ($C_1$-$C_6$)-Alkyl, ($C_2$-$C_6$)-Alkenyl ($C_2$-$C_6$)-Alkinyl, die durch ein- bis neun Halogenatome und/oder ein- bis dreifach durch einen Rest der Gruppe ($C_1$-$C_6$)-Alkoxy, Halo($C_1$-$C_6$)-alkoxy, ($C_1$-$C_6$)-Alkoxycarbonyl, Halo($C_1$-$C_6$)-alkoxycarbonyl, ($C_1$-$C_6$)-Alkylsulfenyl, ($C_1$-$C_6$)-Alkylsulfinyl, Di($C_1$-$C_6$)-Alkylamino, Cyano, Sulfo, Nitro, Hydroxy, Carboxy substituiert sein können;

($C_1$-$C_6$)-Alkylsulfonyl, ($C_1$-$C_6$)-Alkylcarbonyl, Halo($C_1$-$C_6$)-alkylsulfonyl, Halo($C_1$-$C_6$)-alkylcarbonyl, Mono-, Di-($C_1$-$C_4$)-Alkylcarbamoyl,

$R^5$,$R^6$ unabhängig voneinander Halogen, ($C_1$-$C_4$)-Alkyl, ($C_1$-$C_4$)-Alkoxy, ($C_1$-$C_4$)-Alkylcarbonyl, ($C_1$-$C_4$)-Alkoxycarbonyl, ($C_1$-$C_4$)-Alkylsulfenyl, Halo($C_1$-$C_4$)-alkoxy mit ein bis neun Halogenatomen, Di($C_1$-$C_4$)-Alkylamino, Cyano, Sulfo, Nitro,

A,X,Z unabhängig voneinander Sauerstoff, Schwefel,

B Sauerstoff, Schwefel oder Stickstoff, der durch ($C_1$-$C_4$)-Alkyl substituiert sein kann; oder eine Doppelbindung

C,D,E,F,G unabhängig voneinander Kohlenstoff oder Stickstoff

V Kohlenstoff

W ($C_1$-$C_{10}$)-Alkylen Halo($C_1$-$C_{10}$)-Alkylen, die durch ein bis fünf Reste der Gruppe ($C_1$-$C_4$)-Alkyl, Halo-($C_1$-$C_4$)-alkyl, ($C_1$-$C_4$)-Alkoxy oder Halo($C_1$-$C_4$)-alkoxy substituiert sein können,

$Y^1$ H, Amino oder Hydroxy

$Y^2$ Sauerstoff, Schwefel oder Stickstoff, der durch ($C_1$-$C_4$)-Alkyl substituiert sein kann und

n,m unabhängig voneinander die Zahlen 0 bis 4 bedeuten, sowie deren in der Landwirtschaft einsetzbaren oder physiologisch verträglichen Salze.

4. Insektizide, akarizide oder nematozide Mittel, dadurch gekennzeichnet, daß sie eine wirksame Menge einer Verbindung der Formel I oder der Formel Ia enthalten.

5. Verwendung von Verbindungen der Formel I oder der Formel Ia zur Bekämpfung von Schadinsekten, Akariden oder Nematoden.

6. Verfahren zur Bekämpfung von Schadinsekten, Akariden oder Nematoden, dadurch gekennzeichnet,

daß man auf diese oder die von ihnen befallenen Pflanzen, Flächen oder Substrate eine wirksame Menge einer Verbindung der Formel I oder Ia appliziert.

7. Tierarzneimittel, dadurch gekennzeichnet, daß es eine Verbindung der Formel I oder der Formel Ia nach einem oder mehreren der Ansprüche 1-3 enthält oder aus dieser besteht.

8. Mittel zur Bekämpfung von Ekto- und Endoparasiten bei Tieren, dadurch gekennzeichnet, daß es eine Verbindung der Formel I oder der Formel Ia nach einem oder mehreren der Ansprüche 1-3 neben geeigneten Formulierungshilfsmitteln enthält.

9. Verfahren zur Bekämpfung von Ekto- und Endoparasiten bei Tieren, dadurch gekennzeichnet, daß man diese mit einem Mittel gemäß Anspruch 8 behandelt.